(19) Europäisches Patentamt
European Patent Office
Office européen des brevets

(11) Publication number: 0 194 112 B1

(12) **EUROPEAN PATENT SPECIFICATION**

(45) Date of publication of the patent specification: 23.05.90

(51) Int. Cl.5: A61K 31/395, A61K 31/40, A61K 31/415, A61K 31/44, A61K 31/495, A61K 31/535, C07D 205/04, C07D 401/14, C07D 403/06

(21) Application number: 86301449.4

(22) Date of filing: 28.02.86

(54) **3-Aryl-oxyazetidinecarboxamides having anti-muscle tension, anti-muscle spasticity, anticonvulsant and antiepilectic activity.**

(30) Priority: 28.02.85 US 706632
28.02.85 US 706621

(43) Date of publication of application: 10.09.86 Bulletin 86/37

(45) Publication of the grant of the patent: 23.05.90 Bulletin 90/21

(84) Designated Contracting States: AT BE CH DE FR GB IT LI LU NL SE

(56) References cited:
EP-A- 0 102 194
EP-A- 0 102 740
US-A- 4 226 861

(73) Proprietor: A.H. ROBINS COMPANY, INCORPORATED, 1407 Cummings Drive P.O. Box 26609, Richmond Virginia 23261-6609(US)

(72) Inventor: Taylor, Chandler Roy, Jr., 2715 Fenholloway Drive, Mechanicsville Virginia 23111(US)
Inventor: Cale, Albert Duncan, Jr., Route 8 Box 150, Mechanicsville Virginia 23111(US)
Inventor: Johnson, David Norseen, 11406 Blendon Lane, Richmond Virginia 23233(US)
Inventor: Stauffer, Harold Fisher, Jr., 4415 Old Fox Trail, Midlothian Virginia 23113(US)

(74) Representative: Sheard, Andrew Gregory et al, Kilburn & Strode 30, John Street, London WC1N 2DD(GB)

EP 0 194 112 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

This invention relates to 3-aryloxyazetidine carboxamides, certain of which are novel, and their use in the preparation of agents for the treatment or prophylaxis of muscle tension, muscle spasticity, anxiety, convulsions and/or epilepsy in living animals, including humans.

Certain of the compounds useful in the present invention are disclosed in US-A-4226861 as having anticonvulsant activity and use in treating epilepsy having the formula:

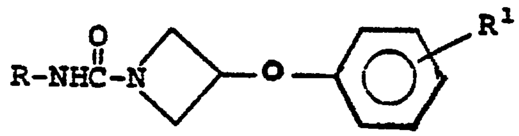

wherein R is loweralkyl and $R^1$ is hydrogen, amino-carbonyl or trifluoromethyl.

Certain other of the compounds useful in the present invention are disclosed in US patent application Serial No. 664,036 filed on October 22, 1984, as having anticonvulsant activity and having the formula:

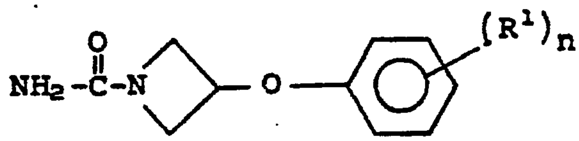

wherein $R^1$ = H, F, loweralkyl, loweralkoxy, trifluoromethyl, acetyl, or aminocarbonyl. That US application is a continuation-in-part application of US application Serial No. 409,476 filed August 19, 1982, which is the priority specification of EP-A 102 194 and which states that the compounds of US-A-4226861, mentioned above, have muscle relaxant property and which; based on another less reliable test, states that the compounds as described in European patent application nr. 83.304 348.2 which has its priority basis in US application No. 409 476 do not have muscle relaxant activity at an effective anticonvulsant dosage. This subject matter subsequently was published in EP-A 0 102 194 on March 7, 1984 and corresponds in essential to the US-patent nr US-A 45 791 393.

EP-A 0 102 740 describes N-formyl and N-hydroxymethyl-3-phenoxy-1-azetidinecarboxamides having anticonvulsant activity.

The 3-aryloxyazetidinecarboxamides useful in the preparation of agents for treating muscle tension, muscle spasticity and/or anxiety in animals of this invention have the formula:

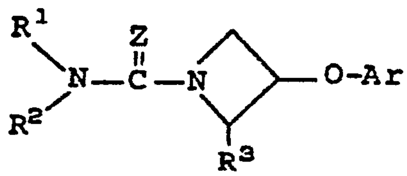

Formula I

wherein Ar represents pyridyl (in any of its positions), halo substituted pyridyl, phenyl, or phenyl substituted by 1 or 2 chloro, bromo, iodo, fluoro, ($C_1$–$C_8$) alkyl, ($C_1$–$C_8$) alkoxy, nitro, aminocarbonyl or trifluoromethyl radicals;
Z represents oxygen or sulphur;
each or $R^1$ and $R^2$ independently represents hydrogen, ($C_1$–$C_8$) alkyl, aryl, allyl, ($C_1$–$C_8$) alkyl substituted allyl, propargyl, cycloalkyl, ($C_1$–$C_8$) alkylcycloalkyl, cycloalkyl ($C_1$–$C_8$) alkyl, aryl ($C_1$–$C_8$) alkyl and di ($C_1$–$C_8$) alkylamino ($C_1$–$C_8$) alkyl, or $R^1$ and $R^2$ when taken together with the adjacent nitrogen atom form a heterocyclic amine radical including azetidinyl, pyrrolidinyl, piperidinyl, homopiperidinyl, imidazolyl, piperazinyl, 4-substituted piperazinyl and morpholinyl;
$R^3$ represents hydrogen, ($C_1$–$C_8$) alkyl, aryl or aryl ($C_1$–$C_8$) alkyl;
or a pharmaceutically acceptable salt thereof when $R^1$ and/or $R^2$ have a salt-forming basic amino component or when Ar is pyridyl and the term "aryl" under the definition of $R^1$, $R^2$ and $R^3$ referring to phenyl or phenyl substituted by non-interfering radicals such as halo, ($C_1$–$C_8$) alkyl, ($C_1$–$C_8$) alkoxy, nitro, cyano, trifluoromethyl, carbomethoxy, and carboethoxy and further the term "arylloweralkyl" under the definition of $R^1$, $R^2$ and $R^3$ referring to an aryl group as defined above linked via 1–8 carbon alkyl chains, including branched chains, to the amide nitrogen.

It will be understood that formula I covers geometrical including <u>cis, trans,</u> (E) and (Z) isomers.

The compounds of Formula I are novel except wherein Ar is phenyl or phenyl substituted by trifluoromethyl or aminocarbonyl at the same time Z is oxygen, $R^3$ is hydrogen, and $R^1$ and $R^2$ are a combination of

hydrogen and ($C_1$–$C_8$) alkyl and wherein Ar is phenyl or phenyl substituted by fluoro, ($C_1$–$C_8$) alkyl, ($C_1$–$C_8$) alkoxy, trifluoromethyl, acetyl or aminocarbonyl at the same time Z is oxygen and $R^1$, $R^2$ and $R^3$ are hydrogen, referred to as compounds of general Formula I'.

The present invention also relates to:

i) pharmaceutical compositions comprising compounds of general Formula I' and a pharmaceutically acceptable carrier therefor;
ii) compounds of general Formula I' for use in human or veterinary medicine;
iii) the use of the compounds of general Formula I' in the preparation of an agent for use in the treatment or prophylaxis of epilepsy and/or convulsions;
iv) a process for the preparation of compounds of general Formula I'; and
v) the use of the compounds of general Formula I in the preparation of an agent for treating muscle tension, muscle spasticity and/or anxiety in animals, including humans, wherein, for the treatment of muscle tension and/or muscle spasticity, the agent is to be administered at a dose of from 4–40 mg/kg per day.

In the further definition of symbols and in the formulae hereof and where they appear elsewhere throughout this specification and in the claims, the terms have the following significance.

The term "loweralkyl" or "($C_1$–$C_8$) alkyl" as used herein, unless otherwise specified, includes straight and branched chain radicals of up to eight carbons inclusive and is exemplified by such groups as methyl, ethyl, propyl, isopropyl, butyl, sec-butyl, tert-butyl, amyl, isoamyl, hexyl, heptyl, and octyl radicals. The term "loweralkoxy" has the formula –0–loweralkyl.

The term "cycloalkyl" as used herein includes primarily cyclic alkyl radicals containing 3–9 carbon atoms inclusive and includes such groups as cyclopropyl, cyclobutyl, cyclo-pentyl, cyclohexyl, and cycloheptyl.

The term "loweralkylcycloalkyl" refers to cycloalkyl substituted by alkyl radicals of 1–8 carbon length.

The term "cycloalkylloweralkyl" refers to a cycloalkyl radical linked via 1–8 carbon alkyl chains, including branched chains, to the amide nitrogen.

The term "substituted allyl" means allyl substituted by alkyl radicals in any one of its 3 positions.

The term "aryl" under the definition of $R^1$, $R^2$ and $R^3$ refers to phenyl or phenyl substituted by non-interfering radicals such as halo, ($C_1$–$C_8$) alkyl, ($C_1$–$C_8$) alkoxy, nitro, cyano, trifluoromethyl, carbomethoxy, or carboethoxy and further the term "arylloweralkyl" under the definition of $R^1$, $R^2$ and $R^3$ refers to an aryl group as defined above linked via 1–8 carbon alkyl chains, including branched chains, to the amide nitrogen.

The term "4-substituted-piperazinyl" under the definition of $R^1$ and $R^2$ refers to a piperazine radical substituted by usual radicals common in the art, including aryl as defined above and loweralkyl as defined above.

As defined above under Formula I, pharmaceutically acceptable salts are included when $R^1$ and/or $R^2$ have a salt-forming amino component or when Ar is pyridyl. Salt-forming amino components are present, for example, when $R^1$ and/or $R^2$ are di($C_1$–$C_8$) alkylamino ($C_1$–$C_8$) alkyl, or when together they form piperazinyl or imidazolyl radicals. Pharmaceutically acceptable acid addition salts are those salts which are physiologically compatible in warm-blooded animals. The acid addition salts may be formed by either strong or weak acids. Representative of strong acids are hydrochloric, hydrobromic, sulfuric, and phosphoric acids. Representative of useful weak acids are fumaric, maleic, succinic, oxalic, citric, tartaric and hexamic.

As stated above, the method of this invention employs the compounds of Formula I to relieve muscle tension and spasticity (i.e., to relax muscles) and/or anxiety in animals, including humans. The procedure for testing compounds for muscle relaxant activity is the Morphine-Induced Straub-Tail-In-Mice Test and the procedure for indicating antianxiety response is the Vogel Conflict Test based on shock suppressed drinking behavior. Both tests are given in detail hereinbelow under "Pharmacological Test Procedures".

The method utilizing one or more of the compounds of Formula I is adaptable for treating tensed and spastic muscles and for treating anxiety in the same individual or for treating tensed and spastic muscles when anxiety is not present or for treating anxiety when there are no muscle problems. Examples of effective muscle treatment would be for muscular conditions arising from tetanus, whiplash, stroke, multiple sclerosis, and cerebral palsy.

Compounds of Formula I wherein $R^1$ and $R^2$ have an allyl, substituted allyl, propargyl or cycloalkyl value are preferred for their potency as muscle relaxants.

The procedure for testing compounds of this invention for their anticonvulsant activity is based on evaluation techniques published by Swinyard, E.A., in EPILEPSIA 10: 107–19 (1969) and in J. PHARMAC. EXPTL. THERAP. 106: 319–30 (1952), and others as explained in greater detail below. The compounds demonstrate protective activity against seizures in both metrazole and electrical challenge and, consequently, are projected to generally have utility in treating both petit and grand mal epilepsy.

The methods used to prepare the muscle relaxants and antianxiety agents of Formula I which are used in the method of this invention are classified as follows by equation under Method Classes A, B, and C.

Method Class A

From Phenoxyazetidine

a)

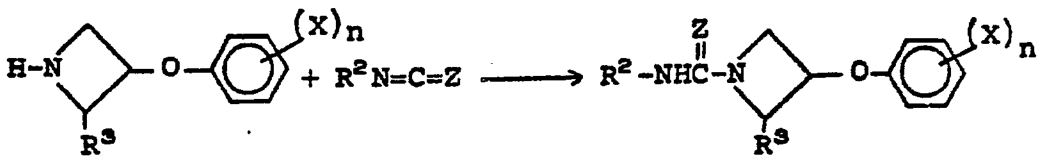

b)

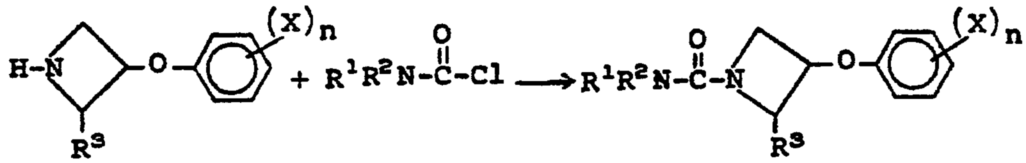

c)

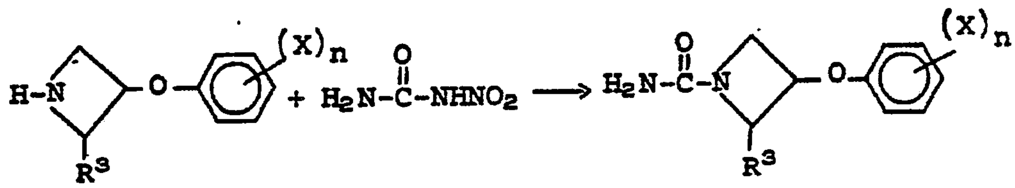

d)

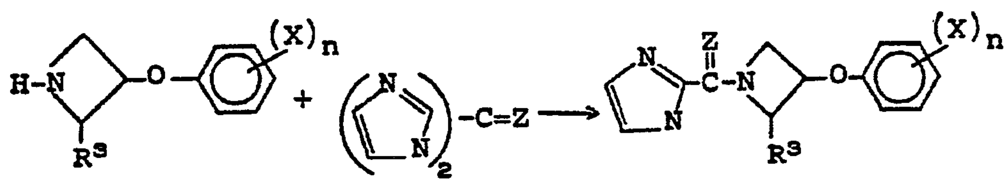

e)

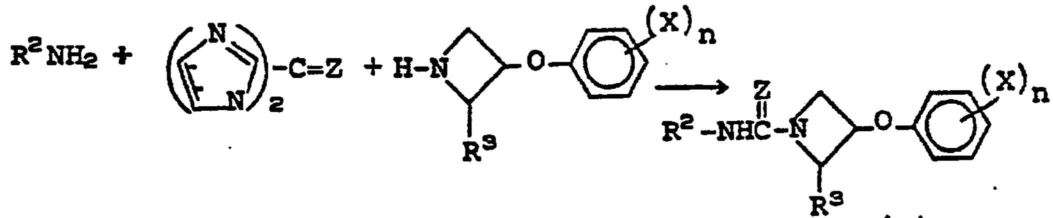

f)

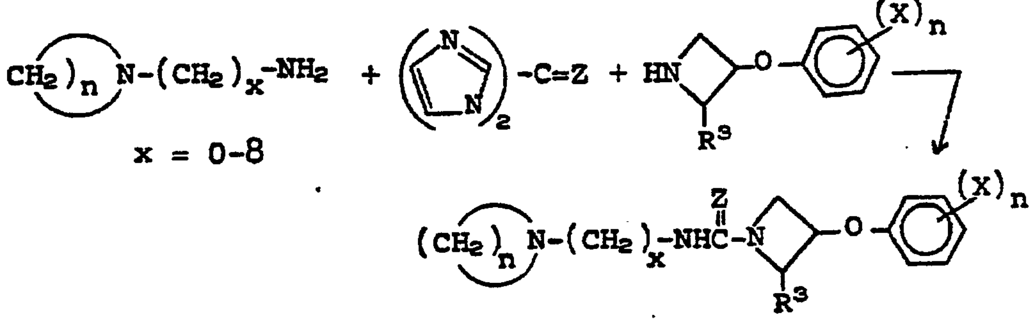

Method Class B

Via Aryloxy-l-chlorocarbonylazetidine
(Ar=Phenyl, Substituted-phenyl, Pyridyl)

a)

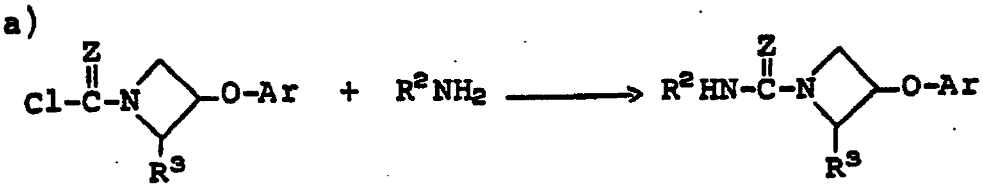

b)

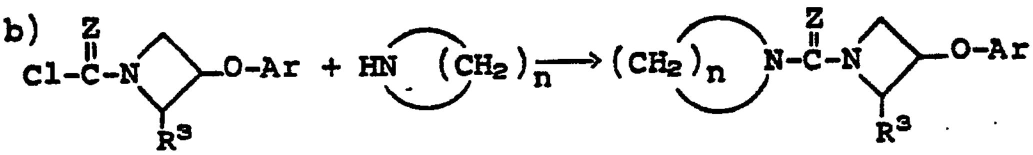

c)

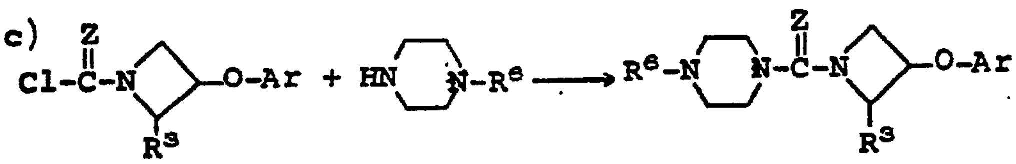

d)

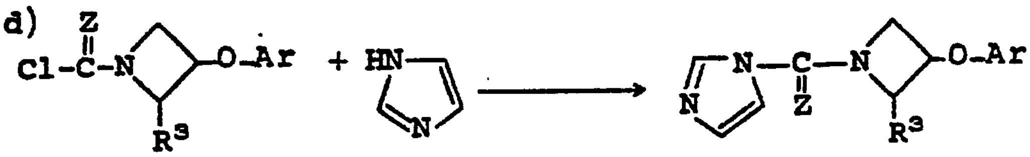

Method Class C

Via Methane (or Phenyl) sulfonyl azetidine

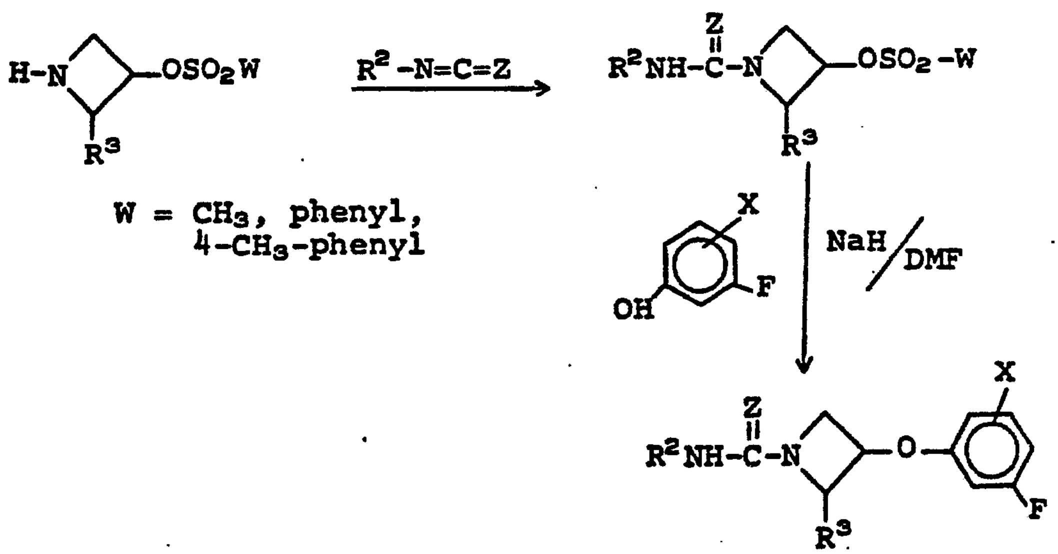


Method Class A reactions comprise a process for preparing phenoxy compounds of Formula I by reacting a compound of the formula:

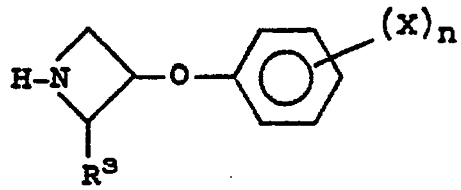

with one of the following classes of compounds:

a) $R^2N = C = Z$

b) 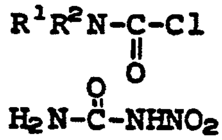

c)

d) 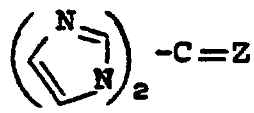

or, e) and f) the product of 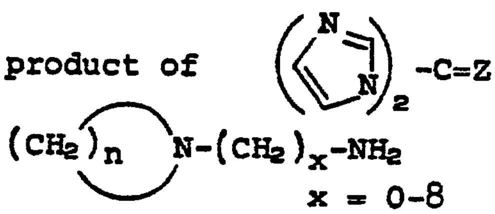

and $R^2NH_2$ or

x = 0-8

Method Class B reactions comprise a process for preparing certain aryloxy compounds of Formula I by reacting a compound of the formula:

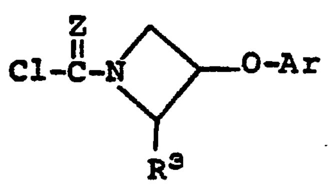

with one of the following classes of compounds:
a) $R^2NH_2$
or, b) a heterocyclic amino compound.

Method Class C reactions comprise a process for preparing m-fluorophenoxy compounds of Formula I by reacting a compound of the formula:

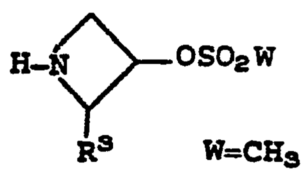

$W = CH_3$, $C_6H_5$, $4\text{-}CH_3\text{-}C_6H_5$

with an isocyanate of the formula

$R^2\text{-}N{=}C{=}Z$

to give a compound of the formula

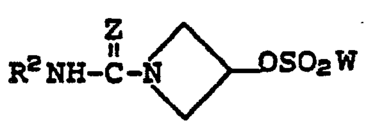

and thereafter reacting with sodium hydride and a metafluorophenol of the formula

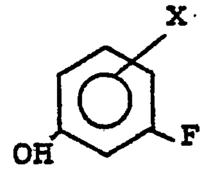

to give a compound of the formula

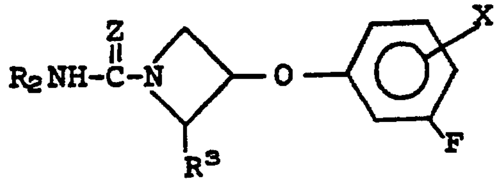

Methods of preparing the starting phenoxyazetidines used in Method Class A are outlined by equation in Chart I. Methods of preparing the starting aryloxy-1-carbonylazetidines used in Method Class B are outlined by equation in Chart II.

The starting 1-(diphenylmethyl)-3-hydroxyazetidines used in Method Class C route (See Chart III) may be prepared by the method of Anderson & Lok., J. ORG. CHEM. (1972) 37: 3953 from benzhydrylamine and an appropriate epihalohydrin. Cis and trans isomers of the 1-(diphenylmethyl)-3-hydroxyazetidines when they exist may be separated by chromatography. Starting α-methylbenzyl-3-azetidinol is prepared by the method of Tetsuya Okutani, et al., CHEM. PHARM. BULL., Vol 22 (1974) p. 1490. Preparations 1-6 illustrate the methods.

Chart I

Preparation of Starting
1-Unsubstituted phenoxyazetidines

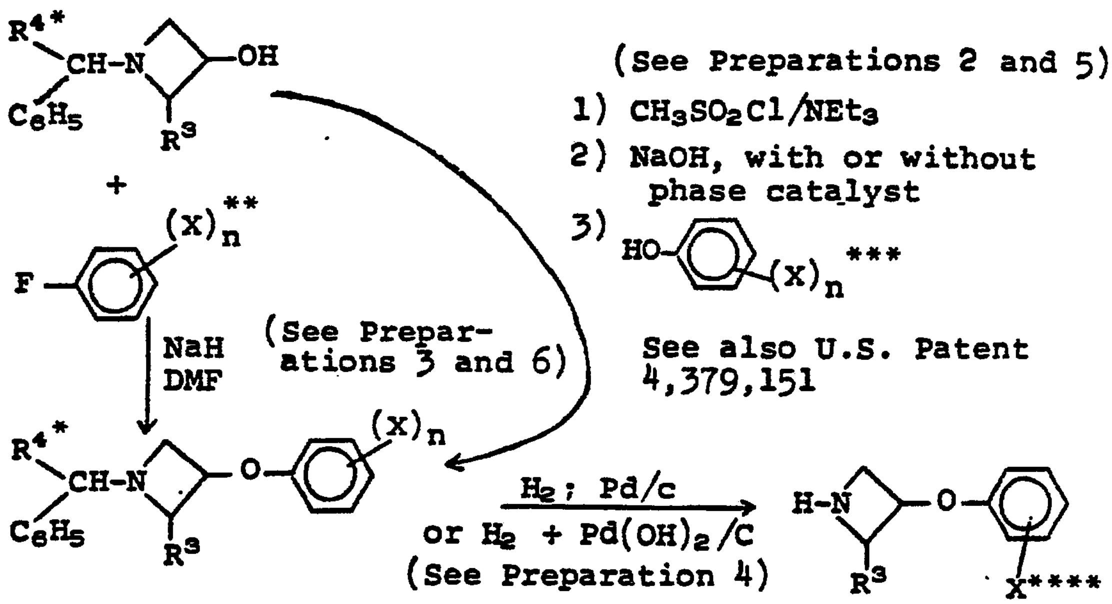


* $R_4 = CH_3$; phenyl or subst. phenyl.
** X is restricted to substituents that are electron withdrawing; e.g., fluoro, chloro, bromo, iodo of $CF_3$.
*** When X = 4-cl, 4-Br, 4-I, 4-F or 4-$CF_3$, yields are very low.
**** Except X cannot be chloro, bromo, or iodo in the product as hydrogenolysis removes these radicals.

Chart II

Preparation of Starting
1-Carbonyl-3-aryloxy azetidines

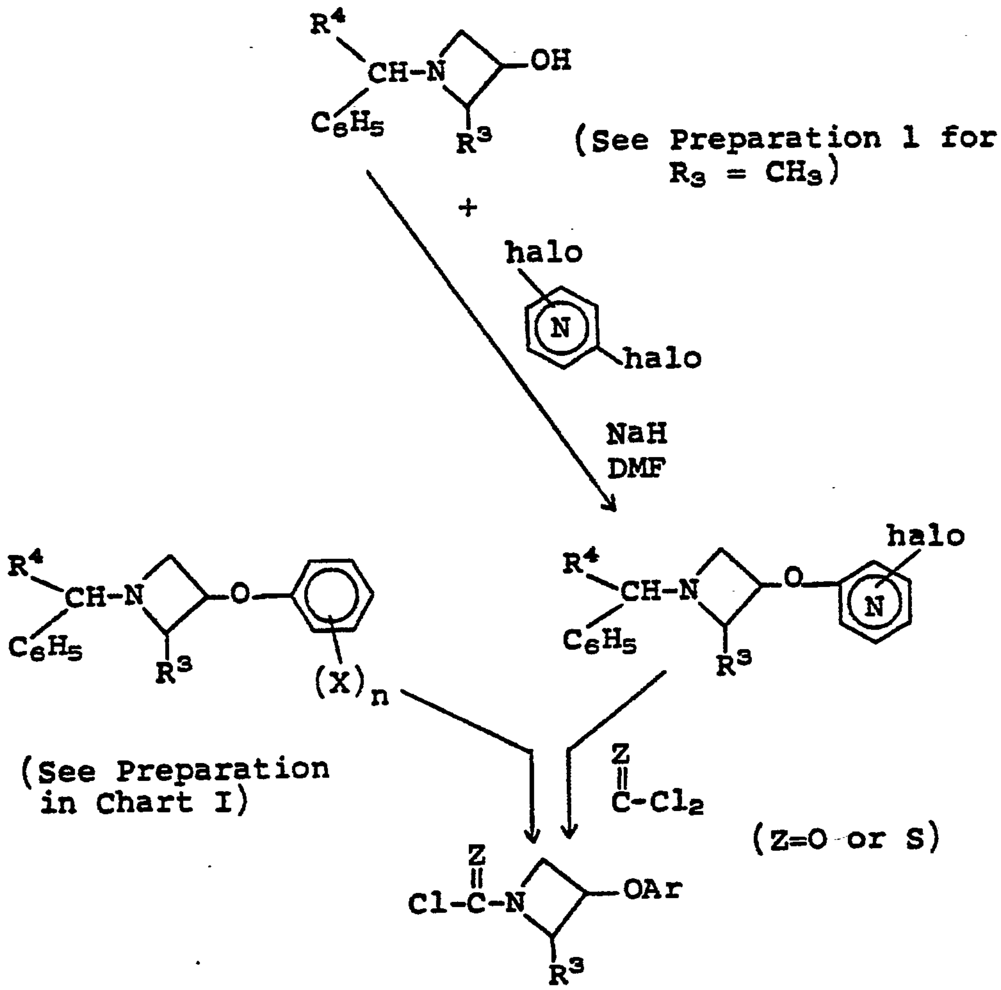

Chart III

Preparation of Starting
Methane-(or phenyl)-sulfonylazetidine

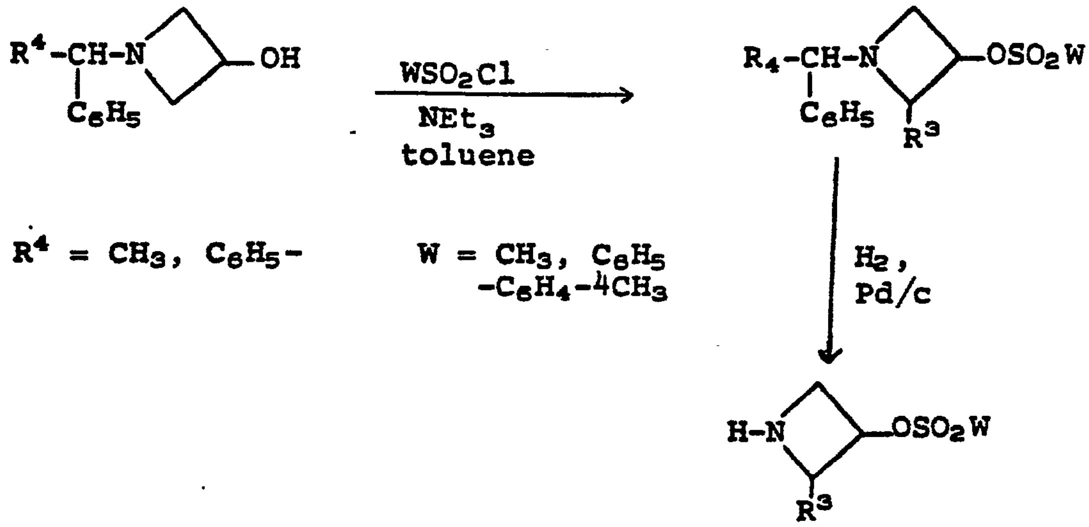


Preparation 1

trans-1-(Diphenylmethyl)-2-methyl-3-azetidinol oxalate [1:1].

A mixture of 126.4 g (0.72 mole) of diphenylmethylamine and 100 g (0.66 mole) of 3-bromo-1,2-epoxybutane in 300 ml of methanol was stirred while being protected from light for 96 hr, then heated at reflux for 30 hr as the color changed from pale yellow to deep amber. A sample was assayed by $^1$H-nmr and showed 3 methyl doublets. A fine beige precipitate was removed by filtration (diphenylmethylamine hydrobromide) and the filtrate concentrated on a rotary evaporator to yield 174.6 g of crude oil. A 1.5 sample was neutralized and placed on a 4 mm thick plate of a Chromatotron® and eluted with 10% ethyl acetate-toluene. A total of sixteen fractions were collected which consisted of 6 distinct spots by TLC. The major component separated was 700 mg and appeared to be the trans isomer. This sample was converted to the oxalate salt. The main concentrate was converted to the free base with ammonium hydroxide and extracted into toluene which was dried over magnesium sulfate and concentrated. The reaction residue was dissolved in methanol and treated with 58 g of oxalic acid, heated to give a homogenous solution and allowed to cool after seeding with a sample of the trans oxalate salt. Filtration yielded 62 g of white granular product, m.p. 147-148.5°C. A second crop of 26 g was also obtained. The $^1$H-nmr spectrum showed only a single $CH_3$ doublet with j ($CH_3$-H) of 6.1 Hz which is consistent with the trans compound.* Total yield of title compound was 88 g (38.8%). * Robert H. Higgins and Norman H. Cromwell, J. Hetero. Chem. 8 (6), 1059-62 (1971).

| | | | | |
|---|---|---|---|---|
| Analysis: | Calculated for $C_{17}H_{19}NO \cdot C_2H_2O_4$: | C, 66.46; | H, 6.16; | N, 4.08 |
| | Found: | C, 66.38; | H, 6.16; | N, 4.07 |

Preparation 2

1-(Diphenylmethyl)-3-azetidinol methanesulfonate (ester) hydrochloride [1:1].

A mixture of 60.02 g (0.22 mole) of 1-diphenylmethyl-3-azetidinol hydrochloride and 48.94 g (0.484 mole) of triethylamine in 800 ml of toluene was stirred for 24 hr, then cooled to 5°C. in an ice bath and treated with 27.7 g (0.24 mole) of methanesulfonyl chloride added at a rate which maintained the temperature below 15°C. The reaction mixture was stirred for 3 hr and filtered to remove the triethylamine hydrochloride. The filtrate was treated with 40 g (0.242 mole) of 4-trifluoromethylphenol followed by 19.35 g (0.484 mole) of sodium hydroxide and 1.6 g (0.005 mole) of tetrabutylammonium bromide in 60 ml of water. The reaction mixture was stirred rapidly at reflux for 18 hrs, then stirred for 72 hr while it cooled to ambient temperature. The reaction mixture was transferred to a separatory funnel and washed with 4 x 200 ml of water (emulsion). The toluene phase was dried over magnesium sulfate and concentrated in vacuo to 82 g of oil. This residue was dissolved in 200 ml of isopropyl alcohol and treated with 20 ml of concentrated hydrochloric acid. Upon cooling, a solid separated and was removed by filtration (5.1 g). The filtrate was treated with isopropyl ether to give an oil which was worked up later. The solid was identified by spectral analysis as the methylsulfonate of the starting azetidinol. Recrystallization from isopropyl alcohol gave 3.3 g of fine white crystalline material, m.p. 172-173°C., (shrinks 167°C.).

| | | | | |
|---|---|---|---|---|
| Analysis: | Calculated for $C_{17}H_{19}NO_3S \cdot HCl$: | C, 57.70; | H, 5.70; | N, 3.96 |
| | Found: | C, 57.80; | H, 5.86; | N, 3.92 |

Preparation 3

trans-1-(Diphenylmethyl)-2-methyl-3-[3-(trifluoromethyl) phenoxy]azetidine oxalate [1:1].

A stirred slurry of 1.2 g (0.03 mole) of sodium hydride (60% dispersion in mineral oil) in 50 ml of dry DMF was treated with 3.45 g (0.01 mole) of trans-1-diphenylmethyl-2-methylazetidin-3-ol oxalate added in small portions. When the addition was complete and the evolution of hydrogen ceased, the reaction was heated to 80°C. for 2 hr then 1.64 g (0.01 mole) of 3-fluoro-trifluoromethylbenzene was added dropwise. The reaction mixture was stirred at 80°C. for an additional 18 hr. The reaction mixture was diluted with ice water and extracted with 3 x 25 ml of toluene. The extracts were combined, dried over magnesium sulfate, filtered, and the filtrate treated with 1 g of oxalic acid. The resulting solid was collected by filtration. Recrystallization from acetone-isopropyl ether yielded 2.2 g (4.5%) of fine white crystals, m.p. 146-147°C. Proton NMR shows it to be the trans compound.

| | | | | |
|---|---|---|---|---|
| Analysis: | Calculated for $C_{24}H_{22}F_3NO \cdot C_2H_2O_4$: | C, 64.06; | H, 4.96; | N, 2.87 |
| | Found: | C, 64.26; | H, 4.99; | N, 2.89 |

Preparation 4

trans-2-Methyl-3-[3-(trifluoromethyl)phenoxy]azetidine oxalate [1:1].

A methanol-warm water solution of 33 g (0.068 mole) of trans-1-diphenylmethyl-2-methyl-3-[3-(trifluoromethyl) phenoxy]azetidine oxalate was treated with ammonium hydroxide until basic, then extracted with 4 x 150 ml of methylene chloride. The combined methylene chloride extracts were washed with water, dried over magnesium sulfate, and concentrated in vacuo to a pale yellow oil. This oil was dissolved in 200 ml of 190 ethanol plus 5 ml of triethylamine and hydrogenated on a Parr apparatus with 3.3 g of 5% palladium-on-charcoal catalyst with a 40 psi atmosphere of hydrogen at 70°C. for 12 hr. After the calculated amount of hydrogen had been absorbed, the catalyst was removed by filtration and the filtrate concentrated in vacuo to yield 26.73 g of crude product. An 8 g portion was converted to the oxalate salt in isopropyl alcohol yielding 6.1 g of fine white powder; m.p. 155-156°C. Total yield extrapolated from the aliquot converted to the oxalate salt was 84% of theory.

| | | | | |
|---|---|---|---|---|
| Analysis: | Calculated for $C_{11}H_{12}F_3NO \cdot C_2H_2O_4$: | C, 48.61; | H, 4.39; | N, 4.36 |
| | Found: | C, 48.67; | H, 4.38; | N, 4.34 |

Preparation 5

trans-1-(Diphenylmethyl)-2-methyl-3-azetidinol methane-sulfonate (ester) hydrochloride [1:1].

A solution of 6 g (0.025 mole) of trans-1-diphenylmethyl-2-methylazetidin-3-ol (obtained from the hydrochloride salt by partitioning in organic solvent and aqueous base, separating and evaporating the organic phase) in 40 ml of dry benzene was treated with 10 ml of triethylamine and cooled to 5°C. While stirring, the reaction mixture was treated with 3.54 g (0.03 mole) of methanesulfonyl chloride at a rate to control the temperature below 10°C. After stirring for 3 hr, TLC (20% ethyl acetate/methylene chloride on silica gel) showed the reaction to be incomplete. An additional 1.14 g (0.01 mole) of methanesulfonyl chloride was added and stirring continued for 1 hr. The reaction mixture was diluted with 100 ml of water and the benzene layer separated, washed with 300 ml of water, dried over magnesium sulfate and concentrated to an oil. The oil was dissolved in isopropyl ether and treated with ethereal hydrogen chloride. The solid salt was removed and recrystallized from 190 ethanol to give 3.4 g (37%) of fluffy white crystals, m.p. 152-153°C.

| | | | | |
|---|---|---|---|---|
| Analysis: | Calculated for $C_{18}H_{21}NO_3S \cdot HCl$: | C, 58.77 | H, 6.03; | N, 3.81 |
| | Found: | C, 58.68; | H, 6.08; | N, 3.80 |

Preparation 6

1-(Diphenylmethyl)-3-[3-(trifluoromethyl)phenoxy] azetidine.

N-Diphenylmethyl-3-hydroxyazetidine hydrochloride (I) was prepared from benzhydrylamine and epichlorohydrin according to Anderson and Lok, J. Org. Chem., 37:3953 (1972). I (41.33 g, 0.15 mole) and triethylamine (42 ml, 0.30 mole) were stirred in toluene (250 ml) while methane sulfonylchloride (12 ml, 0.15 m) was added dropwise over 10 minutes with stirring and the temperature was maintained between 4° and 12°C. TLC (silica gel, 10% ethyl acetate in methylene chloride) at one hour showed all starting materials had reacted. The mixture was filtered to remove the triethylamine hydrochloride which was rinsed twice with toluene. The filtrate and washings combined to about 450 ml of solution. To this solution was added m-trifluoromethyl-phenol(27.5 g, 0.17 mole),tetrabutyl ammonium bromide (2.4 g), 50% sodium hydroxide (24 g, 0.3 mole) and water (24 ml) and the mixture was stirred vigorously and heated to reflux under nitrogen for2.5 hr. The toluene layer of the mixture was separated and washed once with water, dried over sodium sulfate and evaporated to an oil. This oil was seeded and pumped on an oil pump overnight. A solid cake weighing 49.7 g was obtained. Some of this solid was dissolved in isopropanol with brief heating. Water was then added to cause slight cloudiness. The mixture was seeded and cooled to cause crystallization. The white solid was collected by filtration, washed with 50% aqueous isopropanol, and dried under vacuum overnight. Proton NMR showed slight contamination by silicone oil, m.p. 82.5-84°C.

| | | | | |
|---|---|---|---|---|
| **Analysis:** | **Calculated for $C_{23}H_{20}F_3NO$:** | **C, 72.05;** | **H, 5.26;** | **N, 3.65** |
| | **Found:** | **C, 71.62;** | **H, 5.29;** | **N, 3.61** |

The following examples illustrate preparation of compounds encompassed by Formula I and useful in the method of treatment of the invention. The scope of the invention is not limited by the examples, however.

Example 1

N-Methyl-3-[3-(trifluoromethyl)phenoxy]-1-azetidine-carbothioamide.

Crude 3-[3-(trifluoromethyl)phenoxy]azetidine from catalytic debenzylation of 26.0 g (0.078 mole) of 1-benzhydryl-3-[3-(trifluoromethyl)phenoxy]azetidine was dissolved in 100 ml of methylene chloride and treated dropwise under a nitrogen atmosphere with a solution of 5.0 g (0.0678 mole) of methylisothiocyanate in 15 ml of methylene chloride. The reaction mixture was stirred for 16 hr at ambient temperature and let stand over the weekend. The solution was filtered through a celite filter pad to remove a fine crystalline precipitate and the filtrate was evaporated under reduced pressure. The residual oil was crystallized from isopropyl ether to give 12.6 g of product, m.p. 79.86°C. A 5.0 g sample was recrystallized from isopropyl ether (charcoal) to give 3.2 g, m.p. 89.93°C., which was shown by TLC on silica gel (10% methanol-toluene) to be contaminated by a lower $R_f$ material. The filtrate was evaporated under reduced pressure, combined with the 3.2 g of solid and dissolved in 100 ml of methylene chloride. The solution was stirred with 25 g of silica gel for 0.5 hr and filtered through a sintered glass filter. The silica gel was washed with a small volume of methylene chloride and the filtrate evaporated under reduced pressure. The residual solid was recrystallized from isopropyl ether to give 1.3 g of pure product, m.p. 96-98°C.

| | | | | |
|---|---|---|---|---|
| **Analysis:** | **Calculated for $C_{12}H_{13}F_3N_2OS$:** | **C, 49.65;** | **H, 4.51;** | **N, 9.65** |
| | **Found:** | **C, 49.58;** | **H, 4.48;** | **N, 9.58** |

Example 2

N-(2,6-Dimethylphenyl)-3-[3-(trifluoromethyl)phenoxy]-1-azetidinecarbothioamide.

Crude 3-[3-(trifluoromethyl)phenoxy]azetidine from catalytic debenzylation of 30.0 g (0.078 mole) of 1-benzhydryl-3-[3-(trifluoromethyl)phenoxy]azetidine was dissolved in 100 ml of methylene chloride and treated dropwise under a nitrogen atmosphere with a solution of 12.7 g (0.078 mole) of 2,6-dimethylphenylisothiocyanate in 25 ml of methylene chloride. The product began to precipitate during the addition and an additional 50 ml of methylene chloride was added to facilitate stirring. After stirring overnight at ambient temperature, the product was collected by filtratoin (13.5 g, m.p. 196-199°C). A 6.0 g sample was recrystallized from isopropanol to give 5.3 g of product, m.p. 197-199°C.

| | | | | |
|---|---|---|---|---|
| **Analysis:** | **Calculated for $C_{19}H_{19}F_3N_2OS$** | **C, 59.99;** | **H, 5.03;** | **N, 7.36** |
| | **Found:** | **C, 60.04;** | **H, 5.04;** | **N, 7.35** |

Example 3

N-(Phenylmethyl)-3-[3-(trifluoromethyl)phenoxy]-1-azetidinecarboxamide.

To a stirred and chilled (10-20°C.) solution of 0.04 mole of 3-[3-(trifluoromethyl)phenoxy]azetidine in 100 ml of methylene chloride was added dropwise 6.12 g (0.046 mole) of benzyl isocyanate. The reaction mixture was stirred at room temperature for 2 hr and was filtered. The filter cake was washed with petroleum ether (2 x 50 ml), dilute aqueous sodium bicarbonate (2 x 50 ml), and water (2 x 50 ml), yielding 12 g (86%). Recrystallization twice from ethyl acetate gave 9.0 g of clear white flakes, m.p. 173.5-175°C.

| | | | | |
|---|---|---|---|---|
| **Analysis:** | **Calculated for $C_{18}H_{17}F_3N_2O_2$:** | **C, 61.71;** | **H, 4.89;** | **N, 8.00** |
| | **Found:** | **C, 61.57;** | **H, 4.87;** | **N, 7.99** |

Example 4

N-(2,6-Dichlorophenyl)-3-[3-(trifluoromethyl)phenoxy]-1-azetidinecarbothioamide.

A solution of 0.04 mole of 3-[3-(trifluoromethyl) phenoxy]azetidine in 100 ml of absolute ethanol was stirred in a tap water bath while 8.16 g (0.04 mole) of 2,6-dichlorophenyl isothiocyanate was added all at once. The reaction was slightly exothermic and as the isothiocyanate began to dissolve, product began to precipitate. After stirring for 45 minutes the reaction mixture was heated on a steam bath to assure that all the isothiocyanate dissolved, and upon cooling, filtration yielded 15.2 g of white crystalline product. A portion of this material (7.9 g) was recrystallized from absolute ethanol to give 4.3 g of pure crystalline powder, m.p. 196-197°C.

| | | | | |
|---|---|---|---|---|
| **Analysis:** | **Calculated for $C_{17}H_{13}F_3Cl_2N_2OS$:** | **C, 48.47;** | **H, 3.11;** | **N, 6.65** |
| | **Found:** | **C, 48.40;** | **H, 3.07;** | **N, 6.54** |

Example 5

N-[3-(Diethylamino)propyl]-3-[3-(trifluoromethyl) phenoxy]-1-azetidinecarbothioamide, oxalate [1:1].

A solution of (0.0584 mole) of 3-[3-(trifluoromethyl) phenoxy]azetidine was stirred at 10°C. while 10.66 g (0.0584 mole) of 3-(diethylamino)propyl isothiocyanate was added all at once. After stirring overnight at ambient temperature, the reaction mixture was concentrated at 50°C. on a rotary evaporator to a thick syrup residue. The residue was dissolved in isopropanol and treated with 5.3 g of oxalic acid, warmed on a steam bath to dissolve the acid, and upon cooling, a solid salt precipitated. An equal volume of isopropyl ether was added to ensure complete precipitation. Filtration gave 26 g of crude product. A portion (13 g) was recrystallized from isopropanol/ methanol/isopropyl ether (100/50/50) (cooled in a refrigerator) to yield upon filtration 7.5 g of white product, m.p. 155-157°C. Proton NMR confirmed that this was the expected product.

| | | | | |
|---|---|---|---|---|
| **Analysis:** | **Calculated for $C_{18}H_{26}F_3N_3O \cdot C_2H_2O_4$:** | **C, 50.10;** | **H, 5.89;** | **N, 8.76** |
| | **Found:** | **C, 50.02;** | **H, 5.97;** | **N, 8.89** |

Example 6

N-[3-(Dimethylamino)propyl]-3-[3-(trifluoromethyl)-phenoxy]-1-azetidinecarbothioamide.

A stirred solution of 0.0584 mole of 3-[3-(trifluoromethyl)phenoxy]azetidine at 10°C. was treated with 8.42 g (0.0584 mole) of 3-(dimethylamino)propyl isothiocyanate all at once and allowed to stir at ambient temperature overnight. The reaction mixture was treated with 5.3 g (0.0584 mole) of oxalic acid and diluted with 200 ml of isopropyl ether which yielded only 3.8 g of product. The volume was reduced to 100 ml at 50°C. <u>in vacuo</u> and diluted with 500 ml of isopropyl ether to yield an additional 15.3 g of product. The combined solid material was dissolved in isopropyl alcohol and upon cooling, a fine precipitate formed (N,N-dimethyl-1,3-propanediamine oxalate) which was removed by filtration. The product failed to crystallize; addition of isopropyl ether gave only an amorphous gel. After trying to obtain a more satisfactory product for 3 weeks, the reaction material was converted to the free base and taken up in isopropyl ether. The ether solution was stirred with 300 ml of water overnight to remove the diamine. The product crystallized as the free base from the heterogenous mixture and was filtered to give 11.3 g of fine beige crystals. Rework of the filtrate gave an additional 2.3 g of product. A portion (8 g) was recrystallized from benzene/ligroin to yield 5.8 g of very fine beige crystals which were dried at 82°C. under vacuum, m.p. 107-108°C.

| | | | | |
|---|---|---|---|---|
| Analysis: | Calculated for $C_{16}H_{22}F_3N_3OS$: | C, 53.17; | H, 6.14; | N, 11.63 |
| | Found: | C, 53.29; | H, 6.15; | N, 11.60 |

Example 7

N-(2-Propenyl)-3-[3-(trifluoromethyl)phenoxy]-1-azetidinecarboxamide.

A solution of 18.9 g (0.05 mole) of crude 3-[3-(trifluoromethyl)phenoxy]azetidine (contains an equal molar amount of diphenylmethane) in 100 ml of isopropyl ether was stirred under nitrogen while 4.16 g (0.05 mole) of 2-propenyl isocyanate was slowly added. the reaction mixture which was somewhat turbid, cleared and after 1 hr a fine crystalline precipitate began to form. After stirring for 18 hrs, the product was removed by filtration, washed with fresh isopropyl ether and air dried to yield 9.5 g of white crystals, m.p. 75-76°C.

| | | | | |
|---|---|---|---|---|
| Analysis: | Calculated for $C_{14}H_{15}F_3N_2O_2$: | C, 56.00; | H, 5.04; | N, 9.33 |
| | Found: | C, 55.98; | H, 5.05; | N, 9.31 |

Example 8

N-Cyclopropyl-3-[3-(trifluoromethyl)phenoxy]-1-azetidinecarboxamide.

A mixture of 1.9 g (0.033 mole) of cyclopropylamine and 4.9 g of 1,1'-carbonyldiimidazole in 60 ml of tetrahydrofuran was stirred at ambient temperature for 1 hr. The clear solution which formed was treated with a solution of (0.03 mole) of 3-[3-(trifluoromethyl)phenoxy]azetidine in 20 ml of tetrahydrofuran. After stirring overnight, the solid precipitate was removed by filtration to give 4.4 g of gray-white powder. The CI mass spectrum showed a p+1 at 381 m/e which was consistent with the expected product. Recrystallization from benzene/ligroin gave 2.3 g of a light gray powder, m.p. 152-153°C.

| | | | | |
|---|---|---|---|---|
| Analysis: | Calculated for $C_{14}H_{15}F_3N_2O$: | C, 56.00; | H, 5.04; | N, 9.33 |
| | Found: | C, 55.97; | H, 5.07; | N, 9.28 |

Example 9

N-[3-(Diethylamino)propyl]-3-[3-(trifluoromethyl) phenoxy]-1-azetidinecarboxamide oxalate (2:3).

A mixture of 4.3 g (0.033 mole) of 3-diethylamino-propylamine and 4.9 g (0.033 mole) of 1,1'-carbonyldiimidazole in 60 ml of methylene chloride was stirred at ambient temperature for 1 hr. The resulting solution was treated with 3-[3-(trifluoromethyl)phenoxy]azetidine (obtained from 9.21 g (0.03 mole) of the oxalate salt) in 30 ml of methylene chloride. After stirring for 18 hr, the reaction mixture was transferred to a separatory funnel and washed with 3 x 20 ml of water, dried over magnesium sulfate and concentrated in vacuo to a dark oil. The residue (8 g) was chromatographed on a 150 g neutral alumina column by eluting with chloroform. Concentration of the initial fraction gave the product as an amber oil which was dissolved in methyl-isobutyl ketone and treated with 2 g of oxalic acid. Dilution with isopropyl ether gave an oil which solidified and was recrystallized from acetone/isopropyl ether to give 6.25 g (41%) of beige crystals, m.p. 91-93°C.

| | | | | |
|---|---|---|---|---|
| Analysis: | Calculated for $C_{18}H_{26}F_3N_3O_2 \cdot 1.5\ C_2H_2O_4$ | C, 49.61; | H, 5.75; | N, 8.26 |
| | Found: | C, 49.56; | H, 5.73; | N, 8.24 |

Example 10

N-2-(Propenyl)-3-[4-(trifluoromethyl)phenoxy]-1-azetidinecarboxamide.

A solution of 8.7 g (0.04 mole) of crude 3-[4-(trifluoromethyl)phenoxy]azetidine in 75 ml of isopropyl ether was stirred under a blanket of nitrogen while 4.2 g (0.05 mole) of 2-propenyl isocyanate was added dropwise. After stirring for 3 days, no crystalline product precipitated. The reaction mixture was concentrated to a dark reddish oil. TLC (20% ethyl acetate/methylene chloride on silica gel) showed at least 6 spots, all well separated. The residue was dissolved in chloroform, chromatographed on a 350 g silica gel columna and eluted with chloroform until the reddish forerun was removed. The column was then eluted with an ethyl acetate/chloroform gradient to 4% ethyl acetate. All the fractions were combined and concentrated to give 4.8 of orange oil, which crystallized on standing. Recrystallization from acetone/cyclohexane gave 3.3 g (27.5%) of beige crystals, m.p. 91-92.5°C.

| | | | | |
|---|---|---|---|---|
| Analysis: | Calculated for $C_{14}H_{15}F_3N_2O_2$: | C, 56.00; | H, 5.04; | N, 9.33 |
| | Found: | C, 55.98; | H, 5.17; | N, 9.36 |

Example 11

N-(Cyclopropylmethyl)-3-[3-(trifluoromethyl)phenoxy]-1-azetidinecarboxamide.

A solution of 2.6 g (0.024 mole) of (aminomethyl) cyclopropane hydrochloride in 50 ml of pyridine was stirred under a blanket of nitrogen while 3.9 g (0.024 mole) of 1,1'-carbonyldiimidazole was added. After stirring for 45 minutes, the TLC (5% methanol/methylene chloride on silica gel) showed no reaction; therefore, 2 ml of triethylamine was added. The reaction mixture after 10 minutes became cloudy and the TLC showed a new product. The reaction was treated with 6.2 g (0.02 mole) of 3-(3-(trifluoromethyl)phenozy]azetidine oxalate. After stirring for 1 hr, a sample was removed, and upon dilution with water, a solid precipitated. The CI mass spectrum indicated it was product. After 2 days, the reaction was diluted with 5 volumes of water and the resulting precipitate collected by filtration to yield 6.5 g of pale yellow crystalline product. Recrystallization from ethanol/water produced white plate-like crystals which were dried at 82°C. for 3 hr in a drying pistol under vacuum; weight of the product was 5.8 g (92%), m.p. 132-133°C.

| | | | | |
|---|---|---|---|---|
| Analysis: | Calculated for $C_{15}H_{17}F_3N_2O_2$: | C, 57.32; | H, 5.45; | N, 8.91 |
| | Found: | C, 57.22; | H, 5.44; | N, 8.86 |

Example12

N,N-Diethyl-3-[3-(trifluoromethyl)phenoxy]-1-azetidine-carboxamide.

A stirred slurry of 5 g (0.0163 mole) of 3-[3-(trifluoromethyl)phenoxy azetidine oxalate in 50 ml of tetrahydrofuran was treated with 5 ml of triethylamine and after 1 hr, 2.5 g (0.018 mole) of diethylcarbamoyl chloride was added. After stirring an additional 15 hr, the reaction was treated with 10 ml of water and saturated with calcium chloride. The tetrahydrofuran was decanted from the solid residue and concentrated in vacuo to an oil. The crude oil was chroma-tographed on a Water's Prep-LC using 50% ethyl acetate/toluene as the eluent. After concentration of the main fractions 3.1 g (60.1%) of pale yellow oil was obtained.

| | | | | |
|---|---|---|---|---|
| Analysis: | Calculated for $C_{15}H_{19}F_3N_2O_2$: | C, 56.96; | H, 6.05; | N, 8.86 |
| | Found: | C, 56.69; | H, 6.01; | N, 8.77 |

Example 13

N,N-Dimethyl-3-[3-(trifluoromethyl)phenoxy]-1-azetidinecarboxamide.

A stirred slurry of 5 g (0.0163 mole) of 3-[3-(trifluoro-methyl)phenoxy]azetidine oxalate in 50 ml of tetrahydrofuran was treated with 5 ml of triethylamine and after 1 hr, 1.95 g (0.018 mole) of dimethylcarbamoyl chloride was added. After stirring an additional 15 hr, the reaction mixture was treated with 20 ml of water and 10 g of calcium chloride. the tetrahydrofuran layer was decanted and the residue triturated with 20 ml of ethyl acetate, then decanted. The combined tetrahydrofuran and ethyl acetate solution was concentrated in vacuo. The crude residue was chromatographed on a Waters Prep-LC using 50% ethyl acetate/toluene as the eluent. After concentration of the main fractions, 3.6 g (76.6%) of pale yellow oil was obtained.

| | | | | |
|---|---|---|---|---|
| Analysis: | Calculated for $C_{13}H_{15}F_3N_2O_2$: | C, 54.17; | H, 5.25; | N, 9.72 |
| | Found: | C, 53.73; | H, 5.20; | N, 9.60 |

Example 14

N-(2-Propynyl)-3-[3-(trifluoromethyl)phenoxy]-1-azetidinecarboxamide.

A mixture of 3.9 g (0.024 mole) of 1,1'-carbonyl-diimidazole and 1.32 g (0.024 mole) of 2-propynylamine in 50 ml of tetrahydrofuran was stirred at ambient temperature for 1 hr, then treated with 6.2 g of 3-[3-(trifluoromethyl) phenoxy]azetidine. The reaction mixture was treated with 3 ml of triethylamine and stirred for 18 hr. The reaction mixture was diluted with an equal volume of water and filtered to yield 8 g of wet product. Recrystallization from isopropyl ether gave 3.8 g of gray solid, a mixture of product and the

symmetrical urea of starting 2-propynylamine. A second recrystallization from ethanol-water yielded 2.6 g (43.6%) of pure product, m.p. 105-106°C.

| | | | | |
|---|---|---|---|---|
| Analysis: | Calculated for $C_{14}H_{13}F_3N_2O_2$: | C, 56.38; | H, 4.39; | N, 9.39 |
| | Found: | C, 56.32; | H, 4.34; | N, 9.44 |

Example 15

N-Cyclohexyl-3-[3-(trifluoromethyl)phenoxy]-1-azetidinecarboxamide.

A stirred mixture of 5 g (0.0163 mole) of 3-[3-(trifluoromethyl)phenozy]azetidine oxalate and 2.04 g (0.018 mole) of cyclohexyl isocyanate in 50 ml of tetrahydrofuran was treated with 2 ml of triethylamine then stirred for 18 hr. Dilution of the mixture with water gave a solid precipitate which was collected by filtration to yield 12 g of crude product. Recrystallization from acetone/water gave 5 g of fine white crystals, m.p. 148-150°C. TLC (ethyl acetate on silica gel) showed a trace of symmetrical cyclohexyl urea as well as the product. A second recrystallization from isopropanol yielded 1.65 g (29.6%) of white powder; dried under 0.5 mm/Hg vacuum, m.p. 153-154°C.

| | | | | |
|---|---|---|---|---|
| Analysis: | Calculated for $C_{17}H_{21}F_3N_2O_2$: | C, 59.64; | H, 6.18; | N, 8.18 |
| | Found: | C, 59.52; | H, 6.20; | N, 8.17 |

Example 16

N-Cyclopropyl-3-[4-(trifluoromethyl)phenoxy]-1-azetidinecarboxamide.

A stirred slurry of 4.4 g (0.027 mole) of 1,1'-carbonyl-diimidazole in 50 ml of methylene chloride under nitrogen was treated with 1.54 g (0.027 mole) of cyclopropylamine. After a short (2 min) induction period, the clear solution became suddenly exothermic, bringing the reaction to a gentle reflux. After 1 hr when the reaction mixture had cooled to ambient termperature, 9.6 g (0.025 mole) of 3-[4-(trifluoromethyl)-phenoxy]azetidine, 56.66% purity (contains diphenylmethane) was added all at once and stirring continued for 18 hr. The reaction mixture was concentrated on a rotary evaporator to give a partially crystalline residue.The residue was partitioned between 30/60 petroleum ether and water and the resulting waxy solid removed by filtration. Recrystallization from isopropyl ether yielded 5.7 g (75.9%) of silver platelike crystals, m.p. 145-147°C. After drying at 80°C. under 0.5 mm/Hg vacuum, the weight was not diminished, m.p. 152-153°C.

| | | | | |
|---|---|---|---|---|
| Analysis: | Calculated for $C_{14}H_{15}F_3N_2O_2$: | C, 56.00; | H, 5.04; | N, 9.33 |
| | Found: | C, 55.77; | H, 4.98; | N, 9.44 |

Example 17

N-(Cyclopropylmethyl)-3-[4-(trifluoromethyl)phenoxy]-1-azetidinecarboxamide.

A stirred mixture of 4.4 g (0.027 mole) of 1,1'-carbonyldiimidazole and 2.9 g (0.027 mole) of (aminoethyl) cyclopropane hydrochloride in 50 ml of methylene chloride was treated with the dropwise addition of 2.73 g (0.027 mole) of triethylamine. The reaction was exothermic. The mixture was cooled while stirring for 1 hr, then 9.6 g (0.025 mole) of 3-[4-(trifluoromethyl)phenoxy]azetidine 56.66% (contains diphenylmethane) was added all at once and stirring continued for 18 hr. The reaction mixture was concentrated on a rotary evaporator to give an amber residue. Trituration of this residue with 30/60 petroleum ether gave only an insoluble oil. The trituration step was repeated with 2 x 20 ml of 30/60 petroleum ether and the residue treated with water to yield a white solid. The solid was recrystalfrom isopropyl ether to yield 4.8 g (61.8%) of white platelike crystals; after during at 80°C. under 0.5 mm Hg vacuum, m.p. 132-133°C.

| | | | | |
|---|---|---|---|---|
| Analysis: | Calculated for $C_{15}H_{17}F_3N_2O_2$: | C, 57.32; | H, 5.45; | N, 8.91 |
| | Found: | C, 57.26; | H, 5.46; | N, 8.93 |

Example 18

N-[3-(Diethylamino)propyl]-3-[4-(trifluoromethyl) phenoxy]-1-azetidinecarbothioamide.

A stirred solution of 1.92 g (0.005 mole) of 3-[4-(trifluoromethyl)phenoxy]azetidine, 56.66% (contains diphenylmethane) in 20 ml of isopropyl ether was treated with 0.88 g (0.005 mole) of 3-(diethylamino)-propyl isothiocyanate and stirred for 3.5 hr. The reaction mixture was treated with 0.5 g of oxalic acid dissolved in 2 ml of methanol. After stirring for 18 hr, the solid was collected by filtration, yielding 1.9 g of fine tan powder, m.p. 147-150°C. The solid was dissolved in water and treated with dilute sodium hydroxide. An oil separated which solidified and was collected by filtration. Recrystallization from cyclohexane yielded 1.1 g (56.5%) of fine tan crystals, m.p. 109-110°C.

| | | | | |
|---|---|---|---|---|
| Analysis: | Calculated for $C_{18}H_{26}F_3N_3OS$: | C, 55.51 | H, 6.73; | N, 10.79 |
| | Found: | C, 55.68; | H, 6.67; | N, 10.73 |

Example 19

N-[3-(Diethylamino)Propyl]-3-[4-(trifluoromethyl) phenoxy]-1-azetidinecarboxamide oxalate [1:2].

A stirred solution of 4.4 g (0.027 mole) of 1,1'-carbonyldiimidazole in 50 ml of methylene chloride under nitrogen was treated with the dropwise addition of 3.52 g (0.027 mole) of 3-(diethylamino)propylamine. The reaction mixture was stirred for 1 hr as the somewhat exothermic reaction colled to ambient temperature then treated with 9.6 g (0.025 mole) of 3-[4-(trifluoromethyl)phenoxy]azetidine, 56.66% (contains diphenylmethane) all at once. After stirring for 18 hr, the reaction mixture was concentrated on a rotary evaporator and the residue dissolved in toluene. The toluene solution was washed with 3 x 20 ml of water, then treated with 2.5 g of oxalic acid in 10 ml of isopropanol The resulting solid was collected by filtration and triturated with boiling acetone. After filtration, 1.8 g of unidentified fine white precipitate formed which was separated by filtration. the acetone solution was concentrated to a solid which was recrystallized from isopropyl alcohol/isopropyl ether to yield 9.2 g of crude product (4 spots on TLC; 10% methanol/methylene chloride on silica gel). Recrystallization from methyl ethyl ketone yielded 6.8 g (49.1%) of fine white powder, m.p. 129-130°C.

| | | | | |
|---|---|---|---|---|
| Analysis: | Calculated for $C_{18}H_{26}F_3N_3O_2 \cdot 2\ C_2H_2O_4$: | C, 47.74; | H, 5.46; | N, 7.59 |
| | Found: | C, 47.82; | H, 5.68; | N, 7.76 |

Example 20

N-(2-Propynyl)-3-[4-(trifluoromethyl)phenoxy]-1-azetidinecarboxamide.

A solution of 4.4 g (0.027 mole) 1,1'-carbonyl- diimidazole in 50 ml of tetrahydrofuran was stirred under nitrogen while 1.49 g (0.027 mole) of 2-propynylamine was added with a syringe and needle through a septum installed in one neck of the reaction flask. After stirring for 2 hr, 9.6 g (0.025 mole) of 3-[4-(trifluoromethyl)phenoxy]azetidine (56.66% purity; contains diphenylmethane) was added all at once and stirring continued for an additional 18 hr. The reaction mixture was diluted with ice-water and extracted with 30/60 petroleum ether to remove the diphenylmethane. The oily aqueous portion was extracted with 4 x 50 ml of methylene chloride. These extracts were combined, dried over sodium sulfate and concentrated to an amber oil on a rotary evaporator. The oil solidified when triturated with a small amount of isopropyl ether (50 ml). Filtration yielded 6.1 g of rose-tinted solid product. TLC (10% methanol/methylene chloride on silica gel) showed a mixture of 3 products and some starting material. Recrystallization from ethanol/water gave the product in several small fractions. These were combined and recrystallized from isopropyl ether to yield 4.1 g of pale beige powder, m.p. 135-137°C. TLC still showed some symmetrical 2-propynyl urea. The solid was recrystallized again from ethanol/ water to yield 3.5 g (46.9%) of pale yellow crystalline product, m.p. 140-141°C.

| | | | | |
|---|---|---|---|---|
| Analysis: | Calculated for $C_{14}H_{13}F_3N_2O_2$: | C, 56.38; | H, 4.39; | N, 9.39 |
| | Found: | C, 56.34; | H, 4.36; | N, 9.32 |

Example 21

N-(2-Methyl-2-propenyl)-3-[4-(trifluoromethyl)phenoxy]-1-azetidinecarboxamide.

A stirred solution of 3.6 g (0.022 mole) of 1,1'-carbonyldiimidazole in 75 ml of methylene chloride under nitrogen was treated with 1.6 g (0.022 mole) of 2-methyl-2-propenylamine (added via a syringe and needle through a septum placed in one neck of the reaction flask). After stirring for 1 hr, 6.2 g (0.02 mole) of 3-[4-(trifluoromethyl)phenoxy]azetidine oxalate was added all at once followed in 30 min with 5 ml of triethylamine and stirring was continued for 3 hr. The reaction mixture was washed with water (2 x 25 ml), dried over magnesium sulfate and concentrated <u>in vacuo</u>. The oily residue solidified on standing and was recrystallized from isopropyl ether to yield 3.7 g (58.9%) of fine white crystals, m.p. 101-102°C.

| | | | | |
|---|---|---|---|---|
| Analysis: | Calculated for $C_{15}H_{17}F_3N_2O_2$: | C, 57.32; | H, 5.45; | N, 8.91 |
| | Found: | C, 57.45; | H, 5.51; | N, 9.23 |

Example 22

N-(2-Methyl-2-propenyl)-3-[3-(trifluoromethyl)phenoxy]-1-azetidinecarboxamide.

A solution of 3.6 g (0.022 mole) of 1,1'-carbonyldiimidazole in 75 ml of methylene chloride was stirred under nitrogen while 1.6 g (0.022 mole) of methallylamine was added with a syringe and needle through a septum installed in one neck of the reaction flask. The reaction was slightly exothermic. The reaction mixture was stirred for 1 hr, then treated with 6.2 g (0.02 mole) of 3-[3-(trifluoromethyl)phenoxy]azetidine oxalate followed in 0.5 hr with 5 ml of triethylamine and stirring continued for 16 hr. The reaction mixture was washed with 2 x 30 ml of water, dried over magnesium sulfate, and concentrated on a rotary evaporator to yield 6.7 g of oily residue which solidified. The residue was recrystallized from isopropyl ether to yield 5.4 g (85.9%) of fine white crystals, m.p. 90-91°C.

| | | | | |
|---|---|---|---|---|
| Analysis: | Calculated for $C_{15}H_{17}F_3N_2O_2$: | C, 57.32; | H, 5.45; | N, 8.91 |
| | Found: | C, 57.20; | H, 5.50; | N, 8.95 |

Example 23

N-(3-Methyl-2-butenyl)-3-[4-(trifluoromethyl)phenoxy]-1-azetidinecarboxamide.

A solution of 3.6 g. (0.022 mole) of 1,1'-carbonyldiimidazole in 100 ml of methylene chloride was cooled in a tap water bath and while stirring under nitrogen, 1.87 g (0.022 mole) of 3-methyl-2-butenylamine was added dropwise. After stirring for 1 hr, 6.2 g (0.02 mole) of 3-[4-(trifluoromethyl)phenoxy]azetidine oxalate was added all at once followed in 0.5 hr with 5 ml of triethylamine and stirring continued for an additional 16 hr. The reaction mixture was washed with 2 x 50 ml of water, dried over magnesium sulfate and concentrated on a rotary evaporator to yield a semi-solid residue. Trituration with isopropyl ether and filtration yielded 7 g of crude product which was recrystallized from ethanol-water to give 5.5 g (83.8%) of white crystals, m.p. 156.5-158°C.

| | | | | |
|---|---|---|---|---|
| Analysis: | Calculated for $C_{16}H_{19}F_3N_2O_2$: | C, 58.53; | H, 5.83; | N, 8.53 |
| | Found: | C, 58.81; | H, 5.89; | N, 8.58 |

Example 24

N-(3-Methyl-2-butenyl)-3-[3-(trifluoromethyl)phenoxy]-1-azetidinecarboxamide.

A solution of 3.6 g (0.022 mole) of 1,1'-carbonyldiimidazole in 100 ml of tetrahydrofuran was cooled with a tap water bath and stirred under nitrogen while 1.6 g (0.022 mole) of 3-methyl-2-butenylamine was added with a syringe and needle. The reaction mixture was stirred for 1 hr, then treated with 6.2 g (0.02 mole) of 3-[3-(trifluoromethyl)phenoxy]azetidine oxalate followed in 0.5 hr with 5 ml of triethylamine and stirring continued for 72 hr. The reaction mixture was diluted with 500 ml of ice water and extracted with 6 x 50 ml of methylene chloride. The combined extracts were washed with water, dried over magnesium sulfate and concentrated to a solid residue on a rotary evaporator. Recrystallization from ethanol-water yielded 6 g of white crystals, m.p. 143-144°C.

| | | | | |
|---|---|---|---|---|
| Analysis: | Calculated for $C_{16}H_{19}F_3N_2O_2$: | C, 58.53; | H, 5.83; | N, 8.53 |
| | Found: | C, 58.46; | H, 5.86; | N, 8.69 |

Example 25

(E)-N-(2-Butenyl)-3-[4-(trifluoromethyl)phenoxy]-1-azetidinecarboxamide.

A mixture of 3.6 g (0.022 mole) of 1,1'-carbonyldiimidazole and 1.6 g (0.022 mole) of trans-crotylamine was stirred for 1 hr, then treated with 6.2 g (0.02 mole) of 3-[4-(trifluoromethyl)phenoxy]azetidine oxalate and followed in 0.5 hr with 5 ml of triethylamine with stirring continued for 16 hr. The partially crystalline mixture was washed with 2 x 50 ml of water, dried over magnesium sulfate and concentrated on a rotary evaporator to a solid residue, 14.2 g. Recrystallization from methanol-water yielded 5.35 g (85.1%) of fine white crystals, m.p. 157-158°C.

| | | | | |
|---|---|---|---|---|
| Analysis: | Calculated for $C_{15}H_{17}F_3N_2O_2$: | C, 57.32; | H, 5.45; | N, 8.91 |
| | Found: | C, 57.47; | H, 5.49; | N, 9.00 |

Example 26

(E)-N-(2-Butenyl)-3-[3-(trifluoromethyl)phenoxy]-1-azetidinecarboxamide.

A solution of 3.6 g (0.022 mole) of 1,1'-carbonyldiimidazole in 60 ml of methylene chloride was cooled in an ice bath while stirring under nitrogen while 1.6 g (0.022 mole) of trans-crotylamine was added dropwise. After warming to ambient temperature, 6.2 g (0.02 mole) of 3-[3-(trifluoromethyl)phenoxy]azetidine oxalate was added all at once followed in 0.025 hr by 5 ml of triethylamine with stirring continued for 72 hr. The reaction solution was washed with 2 x 50 ml of water, dried over magnesium sulfate and concentrated on a rotary evaporator to a solid residue, 7 g. Recrystallization from methanol-water gave 5.5 g of slightly yellow product. A second recrystallization with charcoal treatment from isopropyl ether yielded 3.75 g (59.7%) of fine white crystals, m.p. 127-128°C.

| | | | | |
|---|---|---|---|---|
| Analysis: | Calculated for $C_{15}H_{17}F_3N_2O_2$: | C, 57.32; | H, 5.45; | N, 8.91 |
| | Found: | C, 57.35; | H, 5.47; | N, 8.94 |

Example 27

N-Phenyl-3-[3-(trifluoromethyl)phenoxy]-1-azetidinecarboxamide.

A stirred slurry of 6.2 g (0.02 mole) of 3-[3-(trifluoromethyl)phenoxy]azetidine oxalate in 60 ml of tetrahydrofuran was treated with 5 ml of triethylamine followed by 2.62 g (0.022 mole) of phenyl isocyanate and stirring continued for 16 hr. The reaction mixture was diluted with water until an oil separated which quickly solidified. The aqueous tetrahydrofuran was decanted and the solid residue recrystallized from ethanol-water to yield 5.3 g (80.1%) of white crystals, m.p. 137-138°C.

| | | | | |
|---|---|---|---|---|
| Analysis: | Calculated for $C_{17}H_{15}F_3N_2O_2$: | C, 60.71; | H, 4.50; | N, 8.33 |
| | Found: | C, 60.81; | H, 4.47; | N, 8.35 |

Example 28

N-Phenyl-3-[4-(trifluoromethyl)phenoxy]-1-azetidinecarboxamide.

A stirred slurry of 6.2 g (0.02 mole) of 3-[4-(trifluoromethyl)phenoxy]azetidine oxalate and 2.62 g (0.022 mole) of phenyl isocyanate in 60 ml of tetrahydrofuran was treated with 5 ml of triethylamine and stirring continued for 16 hr. The reaction mixture was diluted with water until an oil separated. The tetrahydrofuran-water portion was decanted and the residue solidified on standing. Recrystallization from ethanol-water yielded 3.5 g (53.4%) of fine white crystals, m.p. 174.5-176°C.

| | | | | |
|---|---|---|---|---|
| Analysis: | Calculated for $C_{17}H_{15}F_3N_2O_2$: | C, 60.71; | H, 4.50; | N, 8.33 |
| | Found: | C, 60.91; | H, 4.53; | N, 8.35 |

Example 29

trans-N,2-Dimethyl-3-[3-(trifluoromethyl)phenoxy]-1-azetidinecarboxamide.

A stirred solution of 6 g (0.015 mole) of crude trans-2-methyl-3-[3-(trifluoromethyl)phenoxy]azetidine in 50 ml of tetrahydrofuran was treated with 0.94 g (0.0165 mole) of methyl isocyanate added dropwise and stirred for 16 hr under a blanket of nitrogen. Dilution of the reaction mixture with water produced an oil which solidified. After decanting the aqueous tetrahydrofuran phase, the solid residue was recrystallized from ethanol-water to yield 3.95 g (91.4%) of fine white crystals, m.p. 104.5-106°C.

| | | | | |
|---|---|---|---|---|
| Analysis: | Calculated for $C_{13}H_{15}F_3N_2O_2$: | C, 54.17; | H, 5.25; | N, 9.72 |
| | Found: | C, 54.50; | H, 5.29; | N, 9.71 |

Example 30

trans-2-Methyl-3-[3-(trifluoromethyl)phenoxy]-1-azetidinecarboxamide.

A mixture of 6 g (0.015 mole) of crude trans-2-methyl-3-[3-(trifluoromethyl)phenoxy]azetidine (purity 56.6% contains diphenylmethane) and 2.4 g (0.0225 mole) of nitrourea in 40 ml of acetone was treated with 4 ml of water, then heated until a clear homogenous solution was obtained.The reaction mixture was stirred overnight as it cooled to ambient temperature and diluted with water until an oil separated. The oil solidified and was recrystallized from ethanol/water, yielding 4.3 g of white plate-like crystals; m.p. 117-118°C. The product was recrystallized from benzene, yielding 3.35 g (96.8%) of crystals, m.p. 118-119°C.

| | | | | |
|---|---|---|---|---|
| Analysis: | Calculated for $C_{12}H_{13}F_3N_2O_2$: | C, 52.56; | H, 4.78; | N, 10.22 |
| | Found: | C, 52.54; | H, 4.74; | N, 10.17 |

Example 31

trans-2-Methyl-N-(2-propenyl)-3-[3-(trifluoromethyl) phenoxy]-1-azetidinecarboxamide.

A solution of 6 g (0.015 mole) of crude trans-2-methyl-3-[3-(trifluoromethyl)phenoxy]azetidine (56.6%) in 50 ml of tetrahydrofuran was treated with 1.54 g (0.0165 mole) of 2-propenyl isocyanate all at once and stirred under a blanket of nitrogen for 16 hr. The reaction mixture was diluted with water until an oil separated. The oil failed to crystallize and after 7 weeks it was triturated with isopropyl ether (3 x 25 ml). The combined triturates gave 400 mg of white granular crystals (8.5%), m.p. 55-57°C.

| | | | | |
|---|---|---|---|---|
| Analysis: | Calculated for $C_{15}H_{17}F_3N_2O_2$: | C, 57.32; | H, 5.45; | N, 8.91 |
| | Found: | C, 57.36; | H, 5.50; | N, 8.97 |

Example 32

3-(3-Chlorophenoxy)-N-methyl-1-azetidinecarboxamide.

A solution of 1-chlorocarbonyl-3-(3-chlorophenoxy) azetidine (0.01275 mole) in 20 ml of tetrahydrofuran was treated with 4 ml (0.05 mole) of 40% aqueous methylamine and stirred for 16 hr. The reaction mixture was diluted with water until an oil began to separate, then extracted with 3 x 50 ml of benzene. The combined extracts were dried over magnesium sulfate and concentrated to a solid which was recrystallized from benzene/ligroin to yield 1.2 g (40.0%) of fine white crystals, m.p. 140-141°C.

| | | | | |
|---|---|---|---|---|
| Analysis: | Calculated for $C_{11}H_{13}ClN_2O_2$: | C, 54.89; | H, 5.44; | N, 11.64 |
| | Found: | C, 55.05; | H, 5.58; | N, 11.52 |

Example 33

3-(3-Chlorophenoxy)-N-(2-propenyl)-1-azetidinecarboxamide.

A solution of 5.4 g (0.017 mole) of 1-chlorocarbonyl-3-(3-chlorophenoxy)azetidine in 20 ml of tetrahydrofuran was treated with 2.3 g (0.04 mole) of 2-propenylamine and stirred for 2 hr. The reaction solution was concentrated in vacuo to a rose beige solid. Trituration of the solid with water gave, after filtering, 4.4 g of crude product. After drying, the solid was recrystallized with charcoal treatment from 2% acetone/isopropyl ether to yield 1.7 g (37.5%) of pale beige crystals, m.p. 87-89°C.

| | | | | |
|---|---|---|---|---|
| Analysis: | Calculated for $C_{13}H_{15}ClN_2O_2$: | C, 58.54; | H, 5.67; | N, 10.50 |
| | Found: | C, 58.48; | H, 5.72; | N, 10.49 |

Example 34

N-Methyl-3-(2-pyridinyloxy)-1-azetidinecarboxamide.

A 2M benzene solution of phosgene (40 ml, 0.08 mole) was added to a suspension of 10 g of finely ground potassium carbonate in 40 ml of methylene chloride. The mixture was stirred for 15 min. at room temperature and 10 g (0.056 mole of 1-(1-phenylethyl)-3-(2-pyridyloxy)azetidine in 50 ml of methylene chloride was added with mild cooling. The mixture was stirred at room temperature for 1 hr and concentrated on a rotary evaporator (25°C./30 min). The residue was treated with 100 ml of tetrahydrofuran and cooled with an ice bath. To the cooled, stirred mixture was added 20 ml of 40% aqueous methylamine. The

mixture was stirred for 20 min and partitioned between methylene chloride and water. The methylene chloride was dried over sodium sulfate and concentrated. The residue was crystallized from benzene-ethanol and recrystallized from ethyl acetateisopropyl alcohol. Yield of title compound was 2.3 g (14%), m.p. 165-168°C.

| | | | | |
|---|---|---|---|---|
| Analysis: | Calculated for $C_{10}H_{13}N_3O_2$: | C, 57.96; | H, 6.32; | N, 20.28 |
| | Found: | C, 57.93; | H, 6.34; | N, 20.12 |

Example 35

N-(2-Propenyl)-3-(2-pyridinyloxy)-1-azetidinecarboxamide.

To a stirred suspension of 10 g (0.072 mole) of finely ground potassium carbonate in 90 ml of methylene chloride was added 32 ml (0.062 mole) of 2M phosgene in benzene. The mixture was stirred for 15 min and 8 g (0.031 mole) of 1-(1-phenylethyl)-3-(2-pyridyloxy)azetidine in 50 ml of methylene chloride was added. The mixture was stirred at 25°C. for 2 hr and concentrated on a rotary evaporator at 25°C./30 min and the residue was treated with 100 ml of tetrahydrofuran. The stirred mixture was cooled with an ice bath and treated dropwise with 4 g (0.07 mole) of allyl amine. After stirring 30 min at 25°C., the material was partitioned between water and methylene chloride. The methylene chloride was dried and concentrated. The residue was chromatographed on a Waters® PREP-500 HPLC using a silica column and eluting with 50% ethylacetate-hexane. The product was crystallized twice from isopropyl ether. Yield of title compound was 1.5 g (21%), m.p. 72-76°C.

| | | | | |
|---|---|---|---|---|
| Analysis: | Calculated for $C_{12}H_{15}N_3O_2$: | C, 61.79; | H, 6.48; | N, 18.01 |
| | Found: | C, 61.53; | H, 6.50; | N, 17.96 |

Example 36

3-(2-Pyridinyloxy)-1-azetidinecarboxamide.

A 2M benzene solution of phosgene (32 ml, 0.062 mole) was added to a stirred suspension of 10 g of finely ground potassium carbonate in 80 ml of methylene chloride. The mixture was stirred for 15 min and 8 g (0.031 mole) of 1-(1-phenylethyl)-3-(2-pyridyloxy)azetidine in 50 ml of methylene chloride added.The mixture was stirred for 35 min and concentrated on a rotary evaporator (25°C./30 min). The residue was treated with 100 ml of tetrahydrofuran, cooled with an ice bath and 20 ml of concentrated ammonium hydroxide added slowly while stirring vigorously. The mixture was stirred 1 hr at room temperature and partitioned between methylene chloride and water. The water layer was extracted 2 times with methylene chloride and the combined organic layers were concentrated. The residue was crystallized from benzene and recrystallized from isopropyl ether. Yield of title compound was 1.4 g, m.p. 133-137°C.

| | | | | |
|---|---|---|---|---|
| Analysis: | Calculated for $C_9H_{11}N_3O_2$: | C, 55.95; | H, 5.74; | N, 21.75 |
| | Found: | C, 55.73; | H, 5.71; | N, 21.10 |

Example 37

2-[1-(1-Phenylethyl)-3-azetidinyloxy]pyridine.

The maleate salt of 1-(1-phenylethyl)-3-azetidinol (65 g,.22 mole) was partitioned between toluene and dilute sodium hydroxide and was extracted once with toluene. The organic layer was dried (sodium sulfate) and concentrated. The residue was dissolved in 125 ml of dimethylformamide and added dropwise to a stirred suspension of 9.6 g (.24 mole) of sodium hydride (washed three times with isooctane) in 400 ml of dimethylformamide at 25-35°C. The solution was heated to 65°C. and 35 g(.22 mole) of 2-bromopyridine was added dropwise while heating to 75°C. The solution was stirred and heated at 90°C. for 1 hr followed by heating at 120°C. for 2.75 hr. The mixture was stirred at room temperature overnight and concentrated. The residue was partitioned between water and isopropyl ether. The organic layer was washed twice with water, dried (sodium sulfate), filtered and concentrated. Crude yield was 30 g. The residue was distilled to give 8 g, bp 128-134°C./.01 mm of product.

| | | | | |
|---|---|---|---|---|
| Analysis: | Calculated for $C_{16}H_{18}N_2O$: | C, 75.57; | H, 7.13; | N, 11.01 |
| | Found: | C, 75.20; | H, 7.18; | N, 10.90 |

Example 38

1-[3-[4-(Trifluoromethyl)phenoxy]-1-azetidinylcarbonyl]-1H-imidazole.

A mixture of 1.7 g (0.01 mole) 1,1'-carbonyldiimidazole in 50 ml of tetrahydrofuran and 3 g (0.015 mole) of 3-[4-(trifluoromethyl)phenoxy]azetidine was stirred for 6 hr. The reaction mixture was diluted with water and extracted with 3 x 50 ml of methylene chloride. The extracts upon concentrating in vacuo gave an amber residue which was dissolved in 20 ml of benzene and washed with dilute hydrochloric acid, then washed with water. The benzene portion was concentrated to give a semi-solid residue which when triturated with isopropyl ether gave 1.4 g of gray material. Recrystallization from acetonitrile gave 1.3 g (41.8%) of fine gray crystals, m.p. 139-140°C.

| | | | | |
|---|---|---|---|---|
| **Analysis:** | **Calculated for $C_{14}H_{12}F_3N_3O_2$:** | **C, 54.02;** | **H, 3.89;** | **N, 13.50** |
| | **Found:** | **C, 54.33;** | **H, 3.96;** | **N, 13.89** |

Example 39

N-Methyl-3-phenoxy-1-azetidinecarboxamide.

The compound was prepared from the methane sulfonate of 3-phenoxyazetidine and methylisocyanate as described in Example 1 of U.S. Patent, i.e. US-A 4,226,861, m.p. 139-141°C.

Example 40

N-Methyl-3-[4(trifluoromethyl)phenoxy]-1-azetidinecarboxamide.

The compound was prepared from 3-[4-(trifluoromethylphenoxy)azetidine and methylisocyanate as described in Example 3 of U.S. Patent, i.e. US-A 4,226,861, m.p. 154-157°C.

Example 41

N-Methyl-3-[3-(trifluoromethyl)phenoxy]-1-azetidinecarboxamide.

The compound was prepared from 3-[3-(trifluoromethylphenoxy)azetidine and methylisocyanate as described in Example 4 of U.S. Patent, i.e. US-A 4,226,861, m.p. 145-147°C.

Example 42

N-Methyl-3-[2-(trifluoromethyl)phenoxy]-1-azetidinecarboxamide.

The compound was prepared from 3-[2-(trifluoromethyl)-phenoxy)azetidine and methylisocyanate as described in Example 5 of U.S. Patent, i.e. US-A 4,226,861, m.p. 134-136°C.

Example 43

N-Methyl-3-[2-(aminocarbonyl)phenoxy]-1-azetidinecarboxamide.

The compound was prepared from 2-[3-azetidinyloxy) benzamide and methylisocyanate as described in Example 2 of U.S. Patent, i.e. US-A 4,226,861, m.p. 236-240°C.

Example 44

N-Methyl-3-[3-(aminocarbonyl)phenoxy]-1-azetidinecarboxamide.

The compound was prepared from 3-[3-azetidinyloxy) benzamide and methylisocyanate as described in Example 6 of U.S. Patent, i.e. US-A 4,226,861, m.p. 238-240°C.

Example 45

N-Methyl-3-[4-(aminocarbonyl)phenoxy]-1-azetidine-carboxamide.

The compound was prepared from 4-[3-azetidinyloxy) benzamide and methylisocyanate as described in Example 7 of U.S. Patent, i.e. US-A 4,226,861, m.p. 208-210°C.

Example 46

3-[3-(Trifluoromethyl)phenoxy]-1-azetidinecarboxamide.

A mixture of 30.6 g (0.141 mole) of 3-[3-(trifluoromethyl)phenoxy]azetidine and 42 g (0.321 mole) of nitrourea (80%) in 500 ml of acetone was stirred for 5 days (5 days not required, but convenient) at room temperature. The mixture was filtered and the filtrate concentrated in vacuo. The residue was partitioned between 150 ml of water and 100 ml of ethyl acetate and the layers separated. The aqueous layer was washed with 100 ml of ethyl acetate. The ethyl acetate layers were washed with 75 ml of 5% aqueous sodium hydroxide solution followed by 75 ml of water, dried over sodium sulfate and concentrated in vacuo. The residual oil was crystallized from ethyl alcohol-ethyl acetate to give 22 g (60%) substantially the title compound. Recrystallization twice from ethyl alcohol gave 9.9 g of white crystalline solid, m.p. 151-152.5°C.

| | | | | |
|---|---|---|---|---|
| **Analysis:** | **Calculated for $C_{11}H_{11}F_3N_2O_2$:** | **C, 50.77;** | **H, 4.26;** | **N, 10.76** |
| | **Found:** | **C, 50.90;** | **H, 4.29;** | **N, 10.71** |

Example 47

N-Ethyl-3-[3-(trifluoromethyl)phenoxy]-1-azetidinecarboxamide.

To a stirred and chilled (15-20°C) solution of 0.024 mole of 3-[3-(trifluoromethyl)phenoxy]azetidine in 50 ml of dry benzene was added dropwise 1.99 g (0.028 mole) of ethyl isocyanate. The reaction mixture was stirred at room temperature overnight and was diluted with 50 ml of methylene chloride. The solution was washed with 5% sodium hydroxide (2 x 50 ml), water (50 ml), saturated sodium chloride (25 ml), dried over sodium hydroxide, and concentrated in vacuo. he residue (9.6 g) was twice recrystal-lized from ethyl acetate-isopropyl ether to give 5.4 g of a white solid, m.p. 125-126°C.

| | | | | |
|---|---|---|---|---|
| **Analysis:** | **Calculated for $C_{13}H_{15}F_3N_2O_2$:** | **C, 54.16;** | **H, 5.24;** | **N, 9.72** |
| | **Found:** | **C, 54.24;** | **H, 5.23;** | **N, 9.74** |

Example 48

N-(1-Methylethyl)-3-[3-(trifluoromethyl)phenoxy]-1-azetidinecarboxamide.

To a stirred and chilled (10-20°C.) solution of 9.0 g (0.042 mole) of 3-[3-(trifluoromethyl)phenoxy]-azetidine in 100 ml of dry methylene chloride was added dropwise 4.1 g (0.048 mole) of isopropyl isocyanate. The reaction mixture was stirred at room temperature for 2 hr and was diluted with 100 ml of methylene chloride. The solution was washed with 5% sodium hydroxide (2 x 40 ml), water (50 ml), saturated sodium chloride (50 ml), dried(sodium sulfate) and concentrated in vacuo. The residue was crystallized from ethyl acetate, affording 7.6 g (60.6%). Recrystallization from ethyl acetate gave 5.0 g of clear white needles, m.p. 150-151.5°C.

| | | | | |
|---|---|---|---|---|
| **Analysis:** | **Calculated for $C_{14}H_{17}F_3N_2O_2$:** | **C, 55.62;** | **H, 5.68;** | **N, 9.27** |
| | **Found:** | **C, 55.77;** | **H, 5.68;** | **N, 9.22** |

Example 49

N-Propyl-3-[3-(trifluoromethyl)phenoxy]-1-azetidinecarboxamide.

To a stirred and chilled (10-15°C.) solution of 0.027 mole of 3-[3-(trifluoromethyl)phenoxy]azetidine in 100 ml of dry benzene was added dropwise 4.0 g (0.047 mole) of n-propyl isocyanate. The reaction mixture was stirred at room temperature for 30 minutes. The benzene was washed with dilute sodium bicarbonate (50 ml), water (25 ml), saturated sodium chloride (25 ml), and dried(sodium sulfate). The solution volume was reduced to 50 ml, and 30 ml of petroleum ether was added, yielding 6.5 g (81%) of product. Recrystallization from isopropyl ether-isopropyl alcohol gave 6.0 g of small white needles, m.p. 115-117°C.

| | | | | |
|---|---|---|---|---|
| **Analysis:** | **Calculated for $C_{14}H_{17}F_3N_2O_2$:** | **C, 55.63;** | **H, 5.67;** | **N, 9.27** |
| | **Found:** | **C, 55.65;** | **H, 5.68;** | **N, 9.25** |

Example 50

N-Butyl-3-[3-(trifluoromethyl)phenoxy]-1-azetidinecarboxamide.

A solution of 18.9 (0.05 mole) of crude 3-[3-(trifluoromethyl)-phenoxy]azetidine (contains an equal molar amount of diphenylmethane) in 100 ml of isopropyl ether was stirred under nitrogen while 4.96 g (0.05 mole) of N-butyl isocyanate was slowly added. The clear reaction solution became warm to the touch, and after 20 minutes a white crystalline solid began to precipitate. After stirring for 16 hr, the solid was removed by filtration, washed with fresh isopropyl ether and air dried to yield 8 g of pale beige crystals, m.p. 108-109°C.

| | | | | |
|---|---|---|---|---|
| **Analysis:** | **Calculated for $C_{15}H_{19}F_3N_2O_2$:** | **C, 56.96;** | **H, 6.05;** | **N, 8.86** |
| | **Found:** | **C, 56.78;** | **H, 6.06;** | **N, 8.83** |

Example 51

N-Ethyl-3-[4-(trifluoromethyl)phenoxy]-1-azetidinecarboxamide.

A solution of 6.5 g (0.03 mole) of crude 3-[4-(trifluoromethyl)phenoxy]azetidine in 50 ml of isopropyl ether was stirred under a blanket of nitrogen while 2.85 g (0.04 mole) of ethyl isocyanate was added dropwise. After stirring for 2 hr at ambient temperature, a solid began to precipitate, and after 4 hr, the solid was collected by filtration to yield 3.7 g of beige product, m.p. 94-96°C. Rework of the filtrate gave only a trace of additional product. The product was recrystallized from isopropyl ether/hexane (treated with charcoal) to yield 2.61 g (30%) of product, m.p. 109-110°C.

| | | | | |
|---|---|---|---|---|
| **Analysis:** | **Calculated for $C_{13}H_{15}F_3N_2O_2$:** | **C, 54.17;** | **H, 5.25;** | **N, 9.72** |
| | **Found:** | **C, 54.40;** | **H, 5.33;** | **N, 9.89** |

Example 52

N-Butyl-3-[4-(trifluoromethyl)phenoxy-1-azetidinecarboxamide.

A solution of 6.5 g (0.03 mole) of crude 3-[4-(trifluoromethyl)phenoxy]azetidine in 50 ml of isopropyl ether was stirred under a blanket of nitrogen while 4 g (0.04 mole) of n-butyl isocyanate was added dropwise. The reaction was slightly exothermic and after 30 min, a solid separated. The solid was collected by filtration after 3 hr to give 3.85 g of crystalline product, m.p. 135-136°C. After 24 hr, a second batch of crystals was obtained; 1.5 g; m.p. 132-134°C. The two fractions were combined and recrystallized from cyclohexane to yield 3.6 g of product (38%), m.p. 136-137°C.

| | | | | |
|---|---|---|---|---|
| **Analysis:** | **Calculated for $C_{15}H_{19}F_3N_2O_2$:** | **C, 56.96;** | **H, 6.05;** | **N, 8.86** |
| | **Found:** | **C, 57.12;** | **H, 6.13;** | **N, 8.93** |

Example 53

N-Propyl-3-[4-(trifluoromethyl)phenoxy]-1-azetidinecarboxamide.

A solution of 8.7 g (0.04 mole) of crude 3-[4-(trifluoromethyl)phenoxy]azetidine in 75 ml of isopropyl ether was stirred under a blanket of nitrogen while 4.3 g (0.05 mole) of n-propyl isocyanate was added dropwise. After stirring for 2 hr, only a trace of crystalline precipitate formed in the reaction mixture, and after stirring for 18 hr, filtration yielded only 1.1 g of product, m.p. 112-114°C. The filtrate was concentrated *in vacuo* to give a dark amber residue. After 3 days, only 1.3 g of additional crude product could be obtained from isopropyl ether/hexane. All of the reaction products were dissolved in chloroform and chromatographed on a 200 g silica gel column. Elution wth chloroform gave a reddish forerun, which was discarded. The elution was changed to 2% ethyl acetate/chloroform, then to 4% ethyl acetate, and finally to 2% methanol/chloroform. All the fractions were combined and concentrated to yield 4.1 g of white solid. Recrystallization from cyclohexane yielded 2.86 (23.6%) of fine white crystalline product, m.p. 119-120°C.

| | | | | |
|---|---|---|---|---|
| **Analysis:** | **Calculated for $C_{14}H_{17}F_3N_2O_2$:** | **C, 55.63;** | **H, 5.67;** | **N, 9.27** |
| | **Found:** | **C, 55.50;** | **H, 5.77;** | **N, 9.19** |

Example 54

3-[4-(Trifluoromethyl)phenoxy]-1-azetidinecarboxamide.

A solution of 9.6 g (0.025 mole) of 3-[4-(trifluoromethyl)phenoxy]azetidine 56.66% (contains diphenylmethane) in 50 ml of acetone was treated with 4.22 g (0.045 mole) of nitrourea and 5 ml of water. The mixture was heated on a hot plate until a clear solution was obtained then allowed to cool to ambient temperature during the next 4 hr. The reaction mixture was diluted with 200 ml of ice water and an oil separated (diphenylmethane) which was dissolved in 30/60 petroleum ether and separated. Upon standing, a fine white precipitate formed in the aqueous solution. Filtration yielded 3.6 g of fine white crystals, m.p. 176-178°C. After drying under 0.5 mm Hg vacuum at 80°C., the product weight was reduced to 3.1 g (47.7%), m.p. 178-179°C.

| | | | | |
|---|---|---|---|---|
| Analysis: | Calculated for $C_{11}H_{11}F_3N_2O_2$: | C, 50.74; | H, 4.26; | N, 10.77 |
| | Found: | C, 50.72; | H, 4.24; | N, 10.72 |

Example 55

N-(1-Methylethyl)-3-[4-(trifluoromethyl)phenoxy]-1-azetidinecarboxamide.

A stirred slurry of 6.2 g (0.02 mole) of 3-[4-(trifluoromethyl)phenoxy]azetidine oxalate in 60 ml of tetrahydrofuran was treated with 1.8 g (0.022 mole) of 1-methyl-ethyl isocyanate and after 0.5 hr, 5 ml of triethylamine was added. A clear yellow solution was obtained and was stirred for 18 hr, then treated with 10 ml of water, yielding 5.3 g (87.7%) of pale yellow crystals, m.p. 151-152°C.

| | | | | |
|---|---|---|---|---|
| Analysis: | Calculated for $C_{14}H_{17}F_3N_2O_2$: | C, 55.63; | H, 5.67; | N, 9.27 |
| | Found: | C, 55.80; | H, 5.71; | N, 9.24 |

Example 56

N-(1,1-Dimethylethyl)-3-[4-(trifluoromethyl)phenoxy]-1-azetidinecarboxamide.

A stirred slurry of 6.2 g (0.02 mole) of 3-[4-(trifluoromethyl)phenoxy]azetidine oxalate in 60 ml of tetrahydrofuran was treated with 2 g (0.022 mole) of 1,1-dimethylethyl isocyanate and after 0.5 hr 5 ml of triethylamine was added. The reaction slurry quickly turned to a pale yellow solution which was stirred for 18 hr, then treated with 10 ml of water. After 20 min, the tetrahydrofuran portion was separated, dried over magnesium sulfate and concentrated on a rotary evaporator. The solid residue was recystallized from isopropyl ether to yield 5 g (79.0%) of fine white crystals, m.p. 145-146°C.

| | | | | |
|---|---|---|---|---|
| Analysis: | Calculated for $C_{15}H_{19}F_3N_2O_2$: | C, 56.96; | H, 6.05; | N, 8.86 |
| | Found: | C, 56.97; | H, 6.15; | N, 8.86 |

Example 57

N-(2-Methylpropyl)-3-[3-(trifluoromethyl)phenoxy]-1-azetidinecarboxamide.

A stirred solution of 3.6 g (0.022 mole) of 1,1'-carbonyldiimidazole in 100 ml of methylene chloride under nitrogen was treated with 1.6 g (0.022 mole) of 2-methylpropylamine (added via a syringe and needle through a septum placed in one neck of the reaction flask). After stirring for 1 hr, 6.2 g (0.02 mole) of 3-[3-(trifluoromethyl)phenoxy] azetidine oxalate was added all at once followed in 30 min with 5 ml of triethylamine and stirring continued for 18 hr. The reaction mixture was washed with water (2 x 25 ml), dried over magnesium sulfate and concentrated in vacuo, yielding 13 g of crude solid residue. Recrystallization from ethanol/water yielded 5.9 g of product having a pink cast. A second recrystallization from cyclohexane yielded 4.8 g of fine white crystals, m.p. 124-125°C. Rework of the filtrates yielded 1.2 g additional beige crystals. Total yield of product was 75.6% of theory.

| | | | | |
|---|---|---|---|---|
| Analysis: | Calculated for $C_{15}H_{19}F_3N_2O_2$: | C, 56.96; | H, 6.05; | N, 8.86 |
| | Found: | C, 57.01; | H, 6.11; | N, 8.88 |

Example 58

N-(1,1-Dimethylethyl)-3-[3-(trifluoromethyl)phenoxy]-1-azetidinecarboxamide.

A stirred slurry of 6.2 g (0.02 mole) of 3-[3-(trifluoromethyl)phenoxy]azetidine oxalate in 60 ml of tetrahydrofuran was treated with 2 g (0.02 mole) of 1,1-dimethylethyl isocyanate followed in 30 min with 5 ml of triethylamine. The clear solution which developed was stirred for 18 hr. The reaction mixture was treated with 10 ml of water and after 20 min, the tetrahydrofuran portion was separated, dried over magnesium sulfate, and concentrated on a rotary evaporator. The solid residue was recrystallized from ethanol/water, yielding 5.8 g (91.7%) of fine white crystals, m.p. 105-107°C.

| | | | | |
|---|---|---|---|---|
| **Analysis:** | **Calculated for $C_{15}H_{19}F_3N_2O_2$:** | **C, 56.96;** | **H, 6.05;** | **N, 8.86** |
| | **Found:** | **C, 56.95** | **H, 6.14;** | **N, 8.92** |

Example 59

N-(2-Methylpropyl)-3-[4-(trifluoromethyl)phenoxy]-1 azetidinecarboxamide.

A stirred solution of 3.6 g (0.022 mole) of 1,1-carbonyldiimidazole in 100 ml of methylene chloride under nitrogen was treated with 1.6 g (0.022 mole) of 2-methylpropylamine (added via a syringe and needle through a septum placed in one neck of the reaction falsk). After stirring for 1 hr, 6.2 g (0.02 mole) of 3-[4-(trifluoromethyl)phenoxy]azetidine oxalate was added all at once followed in 30 min with 5 ml of triethylamine and stirring was continued for 18 hr. The reaction mixture was washed with water (2 x 25 ml), dried over magnesium sulfate, and concentrated <u>in</u> <u>vacuo</u>. The solid residue was recrystallized from ethanol/water, yielding 5.4 g (85.4%) of pale yellow crystals.

| | | | | |
|---|---|---|---|---|
| **Analysis:** | **Calculated for $C_{15}H_{19}F_3N_2O_2$:** | **C, 56.96;** | **H, 6.05;** | **N, 8.86** |
| | **Found:** | **C, 57.02;** | **H, 6.08;** | **N, 8.82** |

Example 60

3-(3-Chlorophenoxy)-1-azetidinecarboxamide.

A solution of 5.4 g (0.017 mole) of 1-chlorocarbonyl-3-(3-chlorophenoxy)azetidine in 20 ml of tetrahydrofuran was treated with 3 ml of ammonium hydroxide and stirred for 1 hr. The reaction mixture was concentrated <u>in</u> <u>vacuo</u> to a wet solid, 4 g, which was recrystallized after drying, from benzene to yield 1.5 g of white crystalline powder, m.p. 163-164.5°C. Yield calculated from the 1-(2-phenylethyl) azetidine compound was 38.9%.

| | | | | |
|---|---|---|---|---|
| **Analysis:** | **Calculated for $C_{10}H_{11}ClN_2O_2$:** | **C, 52.99;** | **H, 4.89;** | **N, 12.36** |
| | **Found:** | **C, 52.99;** | **H, 4.91;** | **N, 12.32** |

Example 61

3-(3-Fluorophenoxy)-N-methyl-1-azetidinecarboxamide.

A stirred mixture of 5.4 g (0.02 mole) of 3-(3-fluorophenoxy)-azetidine oxalate and 1.7 g (0.022 mole) of methyl isocyanate in 20 ml of tetrahydrofuran was treated with 5 ml of triethylamine then stirring was continued for 3 hr. The reaction was diluted with water and the fine crystalline precipitate obtained was collected by filtration and dried at 60°C. under vacuum to yield 3 g (66.9%) of product, m.p. 155-156°C.

| | | | | |
|---|---|---|---|---|
| **Analysis:** | **Calculated for $C_{11}H_{13}FN_2O_2$:** | **C, 58.92;** | **H, 5.84;** | **N, 12.49** |
| | **Found:** | **C, 58.93;** | **H, 5.91;** | **N, 12.26** |

Example 62

3-(3-Fluorophenoxy)-N-methyl-1-azetidinecarboxamide.

A stirred slurry of 0.38 g (0.02 mole) of 60% sodium hydride as a mineral oil suspension in 10 ml of dry dimethylformamide was treated under nitrogen with the dropwise addition of 1.12 g (0.01 mole) of 3-fluorophenol in 20 ml of dimethylformamide. After 1 hr, the mixture was heated at 90°C. for 20 min then 2.1 g (0.01 mole) of N-methyl-3-[(methanesulfonyl)oxy]-1-azetidinecarboxamide was added as a solid. The reaction mixture was then stirred at 90°C. for 8 hr. The reaction mixture was cooled by adding ice water

then further diluted to 200 ml with water and extracted with 3 x 50 ml of methylene chloride. Less than 1 g of oil was obtained upon concentrations of the extracts. Extraction with 4 x 50 ml of benzene gave only a trace of product. The mass spectrum (CI) showed the expected p+1 at 225 m/e. The product solidified on standing and was recrystallized from methanol/ water to yield 650 mg (29.0%) of flake-like silver crystals, m.p. 156-158°C.

| | | | | |
|---|---|---|---|---|
| Analysis: | Calculated for $C_{11}H_{13}FN_2O_2$: | C, 58.92; | H, 5.84; | N, 12.49 |
| | Found: | C, 58.93; | H, 5.91; | N, 12.42 |

Pharmacological Test Procedures Muscle Relaxant Test

The test procedure relied on to indicate positive muscle relaxant activity is the morphine-Induced Straub Tail in Mice Test described by G. D. Novak in DRUG DEVELOPMENT RESEARCH (1982) 2: 383-386, except 8 animals per group were used per test rather than 10. The test is summarized as follows: The test drug, reference drug, and control articles to be administered are prepared in saline, 0.5% aqueous methylcellulose suspension or other, depending on solubility, in such concentration that the volume administered is 10 ml/kg. The initial screening dose of the test drug is usually 100 mg/kg. Groups of 8 mice are given an IP dose of a compound or vehicle prepared as described above. After 15 min, mice are administered morphine sulfate, 60 mg/kg, subcutaneously. Fifteen minutes after administration of morphine (i.e., 30 min after test compound administration), mice were scored for presence of Straub Tail defined as an elevation of the tail at least 90 degrees from the horizontal. An $ED_{50}$ value may be determined from at least three logarithmically spaced doses by the method of Litchfield and Wilcoxon (1949), J. PHARMACOL. EXP. THER. 96: 99-113. Compared to a reference compound, methocarbamol, which exhibited an $ED_{50}$ of 75-110 in the above Straub Tail Test, the more active compounds of Formula I were 5-10 times more active.

Antianxiety Test

The test screening procedure relied on to indicate positive antianxiety response is a modification of the Vogel Conflict Test which is based on shock-suppressed drinking behavior in rats outlined by J. R. Vogel, et al in PSYCHOPHARMACOLOGY 21: 1-7 (1971). The procedure used is as follows: The test reference and control articles to be administered intraperitoneally in physiological saline, 0.5% aqueous methylcellulose or other depending on a solubility in such concentration that the volume administered is 5 ml/kg. The initial screening dose of the test article is usually 100.0 mg/kg initially, and the reference drug (diazepam) is 5 mg/kg.

Prior to dosing, rats are housed 2 per cage and deprived of water for 48 hr and thereafter randomized into treatment groups of five. Feed is available ad libitum. Thirty minutes after dosing, each rat is placed individually in a plexiglass cage measuring 18 cm in width, 13 cm in height and 29.5 cm in length and equipped with a stainless steel grid floor. The cage is covered with a plastic lid containing holes to facilitate introduction of a water bottle (30 ml plastic centrifuge tube) with a rubber stopper and metal drinking tube. A Drinkometer circuit (Omniteck Electronics, Inc., 3000 Cortona Road, Columbus, Ohio 43204, is connected between the drinking tube and the grid floor of the apparatus so that the rat completes the circuit whenever it licks the tube. The procedure is to allow the rat to find the drinking tube and complete 20 licks (as displayed on the Drinkometer digital readout) prior to the start of the experimental session. Rats not reaching this criterion are discarded. A three minute experimental session is initiated by a 0.25 mA shock at the 20th lick. Rats that continue drinking will experience a shock at each successive 20th lick. The total number of shocks during the experimental session are recorded as follows:

$$\frac{\text{total licks}}{20} + 1 = \text{total shocks}$$

Statistical analysis is performed by the Dunn's Multiple Comparison Test described by O. J. Dunn (1964) TECHNOMETRICS 6(3):241-52. The mean number of shocks experienced by the control group is compared with those of each drug-treated group. Significance is considered at P<0.1. The higher the total shocks compared to control, the more active is the compound. Active compounds may then be similarly tested at reduced dosages. Illustratively, one preferred compound, the compound of Example 16, namely, N-cyclopropyl-3-[4-(trifluoromethyl)phenoxy]-1-azetidinecarboxamide was active at as low a dosage as 10 mg per kg, where total number of shocks taken as calculated above over control was 14/4.6 (P=0.03) compared to about 12-15/4 for diazepam.

Anticonvulsant Pharmacology Activity

Anticonvulsant activity was determined for compounds of Formula I as evidenced by using chemical or electrical challenge as follows.

Metrazole® Chemical Challenge (Swinyard Method)

Groups of 8 adult female mice were randomly assigned to dosage groups according to the method of Steel, R.G.D., and Torrie, J.H. (1960) in "Principles and Procedures of Statistics", McGraw-Hill Book Company, Inc., pp 99-100, pp 428-31. Each mouse was identified with a color code on its tail. The test compounds were administered as solutions or suspensions in 10 ml/kg mouse body weight of 0.5% aqueous methyl cellulose within 15 minutes of preparation of the suspension. Metrazole® (pentylenetetrazol) was prepared as a solution in physiological saline. The mice were not fasted prior to the test. Eight mice were tested at each dosage level.

Each mouse received one dose of the test drug (usually 100 mg/kg for screening) in the 0.5% aqueous methylcellulose or the control article (0.5% aqueous methylcellulose alone) intraperitoneally. Metrazole® (80 mg/kg S.C.) was then given in a loose fold of skin on the back of the neck; i.e., 1/2 hr after the test compound or control article was given. Injections were given with a 1 ml glass tuberculin syringe with appropriate size hypodermic needle (27 gauge for solutions; 23 gauge for suspensions). All injections were given in a volume of 10 ml/kg mouse body weight. Each mouse was observed for 30 minutes following Metrazole® injection. Failure of the animals to exhibit a threshold seizure (a single episode of clonic spasms at least 5 seconds in duration) was defined as protection. Anticonvulsant data were tabulated as the percent protection, i.e,

$$\frac{\text{No. Mice Protected}}{\text{No. Mice Tested}} \times 100.$$

The $ED_{50}$, 95% confidence limits and potency ratio may be ascertained by the computer-based probit analysis ascribed to Finney, D. J. (1964) Statistical Method in Biological Assay, 2nd Ed., New York. Hefner Publishing Co.

Electrical Challenge

Adult female mice in groups of eight were administered the test drug intraperitoneally (usually 100 mg/kg initially for screening) in liquid carrier, usually physiological saline or water. Animals were challenged electrically by placing brass electrodes on the corneas and applying an electrical stimulus (60 Hz, 5 m sec. pulse width, 34 m A intensity) for 0.2 seconds by way of a Grass Stimulator® and constant current unit and a Hunter Timer®. The absence of tonic seizures upon cessation of the stimuli was scored as protection in that animal. The number of animals protected from tonic seizures at a given dose of test drug was determined. The $ED_{50}$, 95% confidence limits and potency ratio may be ascertained by the method of J. T. Litchfield and F. Wilcoxon (1949) J. PHARMACOL. EXP. THER. 96, 99–113.

Some of the more active compounds such as in Examples 7, 8, 10, 14, 16 and 20 exhibit $ED_{50}$'s in the Metrazole® test of 5 to 30 mg/kg and $ED_{50}$'s in the electrical challenge test of 10 to 30 mg/kg.

Pharmaceutical Compositions for Anxiety, Muscle Tension and Spasticity Treatment

The methods of treating anxiety, muscle tension, and spasticity in mammals is best carried out by administering as active ingredients in a pharmaceutical composition at least one of the compounds of Formula I in association with a pharmaceutical carrier or excipient. The compounds are thus presented in a therapeutic composition suitable for oral, rectal, parenteral, subcutaneous, intramuscular, intraperitoneal, or intravenous administration. Thus, for example, the composition for oral administration can take the form of elixirs, capsules, tablets, or coated tablets containing carriers conveniently used in the pharmaceutical art. Suitable tableting excipients include lactose, potato, and maize starches, talc, gelatin, stearic and silicic acids, magnesium stearate and polyvinyl pyrrolidone.

For parenteral administration, the carrier can be comprised of a sterile parenterally acceptable liquid; e.g., water or arachis oil contained in ampoules.

In compositions for rectal administration, the carrier can be comprised of a suppository base, e.g., cocoa butter or glyceride.

Advantageously, the compositions are formulated as dosage units, each unit being adapted to supply a fixed dose of active ingredients. Tablets, coated tablets, capsules, ampoules and suppositories are examples of preferred dosage forms according to the invention. It is only necessary that the active ingredient constitute an effective amount; i.e., such that a suitable effective dosage will be consistent with the dosage form employed. The exact individual dosages as well as daily dosages will, of course, be determined according to standard medical principles under the direction of a physician or veterinarian.

Testing suggests that compounds of Formula I will be effective in man for muscle relaxant effects at 4–40 mg/kg body weight per day. Illustratively, one compound, namely, N-methyl-3-[3-(trifluoromethyl)-phenoxy]-1-azetidine carboxamide (Example 41), was found to be about 5 times as potent as Methocarbamol as a muscle relaxant.

The suggested regimen, for example, for the compound of Example 41 as a muscle relaxant appears to be about 10 mg/kg/day or for a 75 kg individual about a unit dosage of 250 mg, t.i.d.

Effective daily dosages of compounds of Formula I as antianxiety agents are projected to be 1–10 mg/kg/day body weight based on animal data.

Formulation and Administration for Anticonvulsant/Antiepileptic Treatment

The anticonvulsant/antiepileptic 3-phenoxy-1-azetidinecarboxamides of this invention are effective in the treatment of both petit mal epilepsy and grand mal epilepsy. Effective quantities of these compounds may be administered to a living animal body orally as in capsules, tablets or elixirs. It is only necessary that the active ingredient constitute an effective amount, i.e., such that a suitable effective dosage will be obtained consistent with the dosage form employed. The exact individual dosage as well as daily dosages will, of course, be determined according to standard medical principles under the direction of a physician or veterinarian.

Based upon a comparison with known anticonvulsant compounds, daily dosages appear to preferably range from 0.5 to 1.5 milligrams per kilogram of body weight in the treatment of petit mal epilepsy and 25 to 35 milligrams per kilogram of body weight in the treatment of grand mal epilepsy. Very small quantities of the active materials of the present invention, even as low as 0.1 milligram, are effective when minor therapy is involved. Unit dosages are usually 5 milligrams or above and preferably 25, 50 or 100 milligrams per unit dose. The active ingredients of the invention may be combined with other pharmacologically active agents as previously indicated, or with buffers, antacids or the like, for administration and the proportion of the active agent in the composition may be varied widely.

Capsules

Capsules of 5 mg., 25 mg., and 50 mg. of active ingredient per capsule are prepared; with higher amounts of ingredient reduction may be made in the amount of lactose.

| Typical blend for encapsulation | Per Capsule, mg. |
|---|---|
| Active ingredient | 5.0 |
| Lactose | 296.7 |
| Starch | 129.0 |
| Magnesium stearate | 4.3 |
| Total | 435.0 mg. |

Uniformly blend the selected active ingredient with lactose, starch and magnesium stearate and encapsulate the blend.

Additional capsule formulations preferably contain a higher does of active ingredient and are as follows:

| Ingredients | 100 mg. per Capsule | 250 mg. per Capsule | 500 mg. per Capsule |
|---|---|---|---|
| Active ingredient | 100.0 | 250.0 | 500.0 |
| Lactose | 231.5 | 126.5 | 31.1 |
| Starch | 99.2 | 54.2 | 13.4 |
| Magnesium stearate | 4.3 | 4.3 | 5.5 |
| Total, mg. | 435.0 | 435.0 | 550.0 |

Tablets

A typical formulation for a tablet containing 5.0 mg. of active ingredient per tablet follows. The formulation may be used for other strengths of active ingredient by adjustment of weight of dicalcium phosphate.

| Ingredients | Per Tablet, mg. |
|---|---|
| (1) Active ingredient | 5.0 |
| (2) Corn Starch | 13.6 |
| (3) Corn Starch (paste) | 3.4 |
| (4) Lactose | 79.2 |
| (5) Dicalcium phosphate | 68.0 |
| (6) Calcium Stearate | 0.9 |
| Total | 170.1 mg. |

Uniformly blend 1, 2, 4 and 5. Prepare 3 as a 10 percent paste in water. Granulate the blend with the starch paste and pass the wet mass through a number eight mesh screen. The wet granulation is dried and passed through a number twelve mesh screen. The dried granules are blended with calcium stearate and compressed.

Additional tablet formulations preferably contain a higher dosage of the active ingredient and are as follows.

50 mg. Tablet

| Ingredients | Per Tablet, mg. |
|---|---|
| Active ingredient | 50.0 |
| Lactose | 90.0 |
| Corn starch | 58.0 |
| Calcium stearate | 2.0 |
| Total | 200.0 |

Uniformly blend the active ingredient, lactose, and corn starch. The blend is granulated, using water as a granulating medium. The wet granules are passed through an eight mesh screen and dried at 140° to 160°F (60° to 71°C) overnight. The dried granules are passed through a number ten mesh screen and blended with the proper amount of calcium stearate and this blend is then converted into tablets on a suitable tablet press.

## Claims

1. A compound of general formula I':

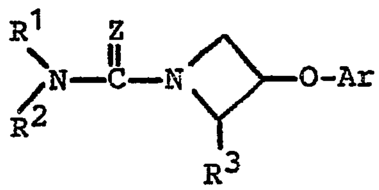

(I')

wherein:
Ar is represents pyridyl (in any of its positions), pyridyl substituted by halo, phenyl or phenyl substituted by 1 or 2 radicals chloro, bromo, iodo, fluoro, ($C_1$–$C_8$) alkyl, ($C_1$–$C_8$) alkoxy, nitro, aminocarbonyl or trifluoromethyl radicals;
Z represents oxygen or sulphur;
each of $R^1$ and $R^2$ independently represents hydrogen, ($C_1$–$C_8$) alkyl, aryl, allyl, ($C_1$–$C_8$) alkyl substituted allyl, propargyl, cycloalkyl, ($C_1$–$C_8$) alkylcycloalkyl, cycloalkyl ($C_1$–$C_8$) alkyl, aryl ($C_1$–$C_8$) alkyl, and di ($C_1$–$C_8$) alkylamino ($C_1$–$C_8$) alkyl, or $R^1$ and $R^2$ when taken together with the adjacent nitrogen atom form a heterocyclic amine radical including azetidinyl, pyrrolidinyl, piperidinyl, homopiperidinyl, imidazolyl, piperazinyl, 4-substituted piperazinyl and morpholinyl;
$R^3$ represents hydrogen, ($C_1$–$C_8$) alkyl, aryl, or a pharmaceutically acceptable acid addition salt thereof when $R^1$ and/or $R^2$ have a salt-forming basic amino component or Ar is pyridyl and the term "aryl" under the definition of $R^1$, $R^2$ and $R^3$ referring to phenyl or phenyl substituted by non-interfering radicals such as halo, ($C_1$–$C_8$) alkyl, ($C_1$–$C_8$) alkoxy, nitro, cyano, trifluoromethyl, carbomethoxy, or carboethoxy and further the term "aryllloweralkyl" under the definition of $R^1$, $R^2$ and $R^3$ referring to an aryl group as defined above linked via 1–8 carbon alkyl chains, including branched chains, to the amide nitrogen;
with the proviso that when $R^3$ represents hydrogen, Z represents oxygen and Ar represents phenyl, or phenyl substituted by trifluoromethyl or by aminocarbonyl, then $R^1$ and $R^2$ cannot be a combination of hydrogen and ($C_1$–$C_8$) alkyl and the further proviso that when $R^3$ represents hydrogen, Z represents oxygen and Ar represents phenyl or phenyl substituted by fluoro, by ($C_1$–$C_8$) alkyl, by ($C_1$–$C_8$) alkoxy, by trifluoromethyl, by acetyl or by aminocarbonyl, then $R^1$ and $R^2$ cannot both be hydrogen.

2. A compound as claimed in claim 1, wherein Ar represents trifluoromethyl-substituted phenyl, 2-pyridinyl or aminocarbonyl-substituted phenyl.

3. A compound as claimed in claim 1 or 2, wherein $R^3$ represents hydrogen or ($C_1$–$C_8$) alkyl.

4. A compound as claimed in claim 1, 2 or 3, wherein at least one of $R^1$ and $R^2$ represents hydrogen.

5. A compound as defined in any one of claims 1 to 4, wherein $R^1$ and/or $R^2$ represent(s) allyl, ($C_1$–$C_8$) alkyl substituted allyl, propargyl or cycloalkyl.

6. N-methyl-3-[3-(trifluoromethyl)phenoxy]-1-azetidinecarbothioamide;
N-(2,6-dimethylphenyl)-3-[3-(trifluoromethyl)phenoxy]-1-azetidinecarbothioamide;
N-(phenylmethyl)-3-[3-(trifluoromethyl)phenoxy]-1-azetidinecarboxamide;
N-(2,6-dichlorophenyl)-3-[3-(trifluoromethyl)phenoxy]-1-azetidinecarbothioamide;
N-[3-(diethylamino)propyl]-3-[3-(trifluoromethyl)phenoxy]-1-azetidinecarbothioamide or a pharmaceutically acceptable acid addition salt thereof;
N-[3-(dimethylamino)propyl]-3-[3-(trifluoromethyl)phenoxy]-1-azetidinecarbothioamide or a pharmaceutically acceptable acid salt thereof;
N-(2-propenyl)-3-[3-(trifluoromethyl)phenoxy]-1-azetidinecarboxamide;
N-cyclopropyl-3-[3-(trifluoromethyl)phenoxy]-1-azetidinecarboxamide;
N-[3-(diethylamino)propyl]-3-[3-(trifluoromethyl)phenoxy]-1-azetidinecarboxamide or a pharmaceutically acceptable acid addition salt thereof;
N-2-(propenyl)-3-[4-(trifluoromethyl)phenoxy]-1-azetidinecarboxamide;
N-(cyclopropylmethyl)-3-[3-(trifluoromethyl)phenoxy]-1-azetidinecarboxamide;
N,N-diethyl-3-[3-(trifluoromethyl)phenoxy]-1-azetidinecarboxamide;
N,N-dimethyl-3-[3-(trifluoromethyl)phenoxy]-1-azetidinecarboxamide;
N-(2-propynyl)-3-[3-(trifluoromethyl)phenoxy]-1-azetidinecarboxamide;
N-cyclohexyl-3-[3-(trifluoromethyl)phenoxy]-1-azetidinecarboxamide;
N-cyclopropyl-3-[4-(trifluoromethyl)phenoxy]-1-azetidinecarboxamide;
N-(cyclopropylmethyl)-3-[4-(trifluoromethyl)phenoxy]-1-azetidinecarboxamide;
N-[3-(diethylamino)propyl]-3-[4-(trifluoromethyl)phenoxy]-1-azetidinecarbothioamide or a pharmaceutically acceptable acid addition salt thereof;
N-(3-diethylamino)propyl]-3-[4-(trifluoromethyl)phenoxy]-1-azetidinecarboxamide or a pharmaceutically acceptable acid addition salt thereof;
N-(2-propynyl)-3-[4-(trifluoromethyl)phenoxy]-1-azetidinecarboxamide;
N-(2-methyl-2-propenyl)-3-[4-(trifluoromethyl)phenoxy]-1-azetidinecarboxamide;
N-(2-methyl-2-propenyl)-3-[3-(trifluoromethyl)phenoxy]-1-azetidinecarboxamide;
N-(3-methyl-2-butenyl)-3-[4-(trifluoromethyl)phenoxy]-1-azetidinecarboxamide;
N-(3-methyl-2-butenyl)-3-[3-(trifluoromethyl)phenoxy]-1-azetidinecarboxamide;
(E)-N-(2-butenyl)-3-[4-(trifluoromethyl)phenoxy]-1-azetidinecarboxamide;
(E)-N-(2-butenyl)-3-[3-(trifluoromethyl)phenoxy]-1-azetidinecarboxamide;
N-phenyl-3-[3-(trifluoromethyl)phenoxy]-1-azetidinecarboxamide;
N-phenyl-3-[4-(trifluoromethyl)phenoxy]-1-azetidinecarboxamide;
trans-N,2-dimethyl-3-[3-(trifluoromethyl)phenoxy]-1-azetidinecarboxamide;
trans-2-methyl-3-[3-(trifluoromethyl)phenoxy]-1-azetidinecarboxamide;
trans-2-methyl-N-(2-propenyl)-3-[3-(trifluoromethyl)phenoxy]-1-azetidinecarboxamide;
3-(3-chlorophenoxy)-N-methyl-1-azetidinecarboxamide;
3-(3-chlorophenoxy)-N-(2-propenyl)-1-azetidinecarboxamide;
N-methyl-3-(2-pyridinyloxy)-1-azetidinecarboxamide or a pharmaceutically acceptable acid addition salt thereof;
N-(2-propenyl)-3-(2-pyridinyloxy)-1-azetidinecarboxamide or a pharmaceutically acceptable acid addition salt thereof;
3-(2-pyridinyloxy)-1-azetidinecarboxamide or a pharmaceutically acceptable acid addition salt thereof;
2-[1-(1-phenylethyl)-3-azetidinyloxy]pyridine or a pharmaceutically acceptable acid addition salt thereof;
1-[3-[4-(trifluoromethyl)phenoxy]-1-azetidinyl carbonyl]-1H-imidazole or a pharmaceutically acceptable acid addition salt thereof;
3-(3-chlorophenoxy)-N-methyl-1-azetidinecarboxamide; or
3-(3-fluorophenoxy)-N-methyl-1-azetidinecarboxamide.

7. A pharmaceutical composition comprising a compound as defined in any of claims 1 to 6 and a pharmaceutically acceptable carrier therefor.

8. A compound as defined in any of claims 1 to 6 for use in human or veterinary medicine.

9. The use of a compound as defined in any of claims 1 to 6 in the preparation of an agent for use in the treatment or prophylaxis of epilepsy and/or convulsions.

10. A process for the preparation of a compound as defined in any of claims 1 to 6;
the process comprising either:
(A) reacting a compound of general formula (II)

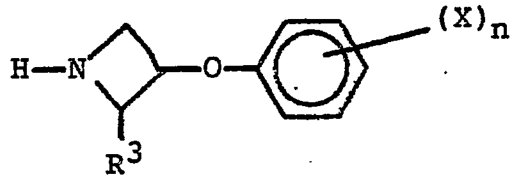

(II)

with one of the following classes of compounds:

a) $R^2N = C = Z$

b) $R^1R^2N-C(=O)-Cl$

c) $H_2N-C(=O)-NHO_2$

d)

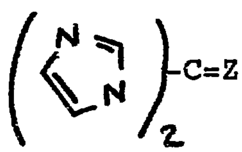

e) and f) the product of

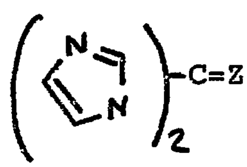

and $R^2NH_2$ or

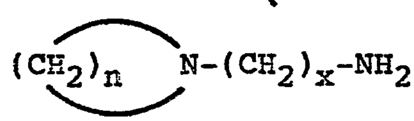

wherein R¹, R², R³, Ar and Z are as defined for general formula I′, $(X)_n$ represents one or more substituents for phenyl as defined for general formula I′ and x is an integer from 0 to 8; or

(B) in the case of certain aryloxy compounds of general formula I′ by reacting a compound of general formula (III)

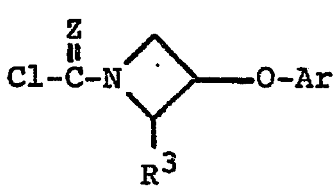

(III)

with one of the following classes of compounds:

a) $R^2NH_2$

b) heterocyclic amino compounds

wherein $R^2$, $R^3$, Ar and Z are as defined for general formula I′; or

(C) in the case of m-fluorophenoxy compounds of general formula I′ by reacting a compound of general formula (IV)

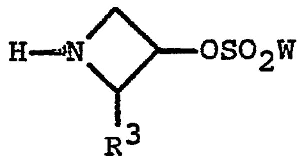

(IV)

wherein $R^3$ is as defined for general formula I′ and W represents methyl, phenyl or 4-methylphenyl with an isocyanate of the formula

$R^2-N=C=Z$

to give a compound of the formula

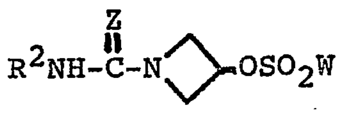

and thereafter reacting with sodium hydride and a metafluorophenol of the formula

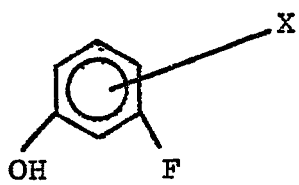

to give a compound of the formula

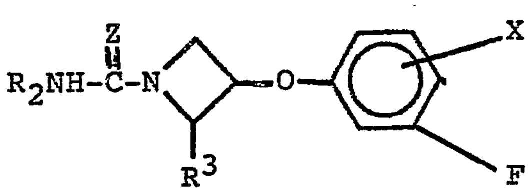

wherein $R^2$, $R^3$, and Z are as defined for general formula I′ and X represents one or more substituents as defined for general formula I′

11. The use of a compound of general formula I:

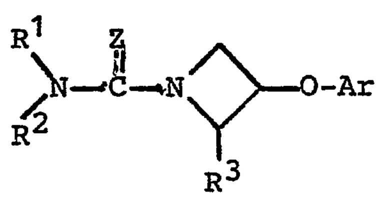

(I)

wherein:

Ar represents pyridyl (in any of its positions), pyridyl substituted by halo, phenyl or phenyl substituted by 1 or 2 radicals chloro, bromo, iodo, fluoro, ($C_1$–$C_8$) alkyl, ($C_1$–$C_8$) alkoxy, nitro, aminocarbonyl or trifluoromethyl radicals;

Z represents oxygen or sulphur;

each of $R^1$ and $R^2$ independently represents hydrogen, ($C_1$–$C_8$) alkyl, aryl, allyl, ($C_1$–$C_8$) alkyl substituted allyl, propargyl, cycloalkyl, ($C_1$–$C_8$) alkylcycloalkyl, cycloalkyl ($C_1$–$C_8$) alkyl, aryl ($C_1$–$C_8$) alkyl, and di ($C_1$–$C_8$) alkylamino ($C_1$–$C_8$) alkyl, or $R^1$ and $R^2$ when taken together with the adjacent nitrogen atom form a heterocyclic amine radical including azetidinyl, pyrrolidinyl, piperidinyl, homopiperidinyl, imidazolyl, piperazinyl, 4-substituted piperazinyl and morpholinyl;

$R^3$ represents hydrogen, ($C_1$–$C_8$) alkyl, aryl, or a pharmaceutically acceptable acid addition salt thereof when $R^1$ and/or $R^2$ have a salt-forming basic amino component or Ar is pyridyl;

in the preparation of an agent for treating muscle tension, muscle spasticity and/or anxiety in animals, including humans, wherein for the treatment of muscle tension and/or muscle spasticity, the agent is to be administered at a dose of from 4–40 mg/kg per day.

12. The use of a compound according to claim 11, wherein Ar represents trifluoromethyl-substituted phenyl, 2-pyridinyl or aminocarbonyl-substituted phenyl.

13. The use of a compound according to claim 11 or 12, wherein $R^3$ represents hydrogen or ($C_1$–$C_8$) alkyl.

14. The use of a compound according to claims 11, 12 or 13, wherein at least one of $R^1$ and $R^2$ represents hydrogen.

15. The use of a compound according to claim 11 which is N-methyl-3-[3-(trifluoromethyl)phenoxy]-1-azetidinecarbothioamide;
N-(2,6-dimethylphenyl)-3-[3-(trifluoromethyl)phenoxy]-1-azetidinecarbothioamide;
N-(phenylmethyl)-3-[3-(trifluoromethyl)phenoxy]-1-azetidinecarboxamide;
N-(2,6-dichlorophenyl)-3-[3-(trifluoromethyl)phenoxy]-1-azetidinecarbothioamide;
N-[3-(diethylamino)propyl]-3-[3-(trifluoromethyl)phenoxy]-1-azetidinecarbothioamide or a pharmaceutically acceptable acid addition salt thereof;
N-[3-(dimethylamino)propyl]-3-[3-(trifluoromethyl)phenoxy]-1-azetidinecarbothioamide or a pharmaceutically acceptable acid addition salt thereof;
N-(2-propenyl)-3-[3-(trifluoromethyl)phenoxy]-1-azetidinecarboxamide;
N-cyclopropyl-3-[3-(trifluoromethyl)phenoxy]-1-azetidinecarboxamide;
N-[3-(diethylamino)propyl]-3-[3-(trifluoromethyl)phenoxy]-1-azetidinecarboxamide or a pharmaceutically acceptable acid addition salt thereof;
N-2-(propenyl)-3-[4-(trifluoromethyl)phenoxy]-1-azetidinecarboxamide;
N-(cyclopropylmethyl)-3-[3-(trifluoromethyl)phenoxy]-1-azetidinecarboxamide;
N,N-diethyl-3-[3-(trifluoromethyl)phenoxy]-1-azetidinecarboxamide;
N,N-dimethyl-3-[3-(trifluoromethyl)phenoxy]-1-azetidinecarboxamide;
N-(2-propynyl)-3-[3-(trifluoromethyl)phenoxy]-1-azetidinecarboxamide;
N-cyclohexyl-3-[3-(trifluoromethyl)phenoxy]-1-azetidinecarboxamide;
N-cyclopropyl-3-[4-(trifluoromethyl)phenoxy]-1-azetidinecarboxamide;
N-(cyclopropylmethyl)-3-[4-(trifluoromethyl)phenoxy]-1-azetidinecarboxamide;
N-[3-(diethylamino)propyl]-3-[4-(trifluoromethyl)phenoxy]-1-azetidinecarbothioamide or a pharmaceutically acceptable acid addition salt thereof;
N-[3-(diethylamino)propyl]-3-[4-(trifluoromethyl)phenoxy]-1-azetidinecarboxamide or a pharmaceutically acceptable acid addition salt thereof;
N-(2-propynyl)-3-[4-(trifluoromethyl)phenoxy]-1-azetidinecarboxamide;
N-(2-methyl-2-propenyl)-3-[4-(trifluoromethyl)phenoxy]-1-azetidinecarboxamide;
N-(2-methyl-2-propenyl)-3-[3-(trifluoromethyl)phenoxy]-1-azetidinecarboxamide;
N-(3-methyl-2-butenyl)-3-[4-(trifluoromethyl)phenoxy]-1-azetidinecarboxamide;
N-(3-methyl-2-butenyl)-3-[3-(trifluoromethyl)phenoxy]-1-azetidinecarboxamide;
(E)-N-(2-butenyl)-3-[4-(trifluoromethyl)phenoxy]-1-azetidinecarboxamide;
(E)-N-(2-butenyl)-3-[3-(trifluoromethyl)phenoxy]-1-azetidinecarboxamide;
N-phenyl-3-[3-(trifluoromethyl)phenoxy]-1-azetidinecarboxamide;
N-phenyl-3-[4-(trifluoromethyl)phenoxy]-1-azetidinecarboxamide;
trans-N,2-dimethyl-3-[3-(trifluoromethyl)phenoxy]-1-azetidinecarboxamide;
trans-2-methyl-3-[3-(trifluoromethyl)phenoxy]-1-azetidinecarboxamide;
trans-2-methyl-N-(2-propenyl)-3-[3-(trifluoromethyl)phenoxy]-1-azetidinecarboxamide;
3-(3-chlorophenoxy)-N-methyl-1-azetidinecarboxamide;
3-(3-chlorophenoxy)-N-(2-propenyl)-1-azetidinecarboxamide;
N-methyl-3-(2-pyridinyloxy)-1-azetidinecarboxamide or a pharmaceutically acceptable acid addition salt thereof;
N-(2-propenyl)-3-(2-pyridinyloxy)-1-azetidinecarboxamide or a pharmaceutically acceptable acid addition salt thereof;
3-(2-pyridinyloxy)-1-azetidinecarboxamide or a pharmaceutically acceptable acid addition salt thereof;
2-[1-(1-phenylethyl)-3-azetidinyloxy]pyridine or a pharmaceutically acceptable acid addition salt thereof;
1-[3-[4-(trifluoromethyl)phenoxy]-1-azetidinylcarbonyl]-1H-imidazole or a pharmaceutically acceptable acid addition salt thereof;
N-methyl-3-phenoxy-1-azetidinecarboxamide;
N-methyl-3-[4-(trifluoromethyl)phenoxy]-1-azetidinecarboxamide;
N-methyl-3-[3-(trifluoromethyl)phenoxy]-1-azetidinecarboxamide;
N-methyl-3-[2-(trifluoromethyl)phenoxy]-1-azetidinecarboxamide;
N-methyl-3-[2-(aminocarbonyl)phenoxy]-1-azetidinecarboxamide;
N-methyl-3-[3-(aminocarbonyl)phenoxy]-1-azetidinecarboxamide;
N-methyl-3-[4-(aminocarbonyl)phenoxy]-1-azetidinecarboxamide;
3-[3-(trifluoromethyl)phenoxy]-1-azetidinecarboxamide;
N-ethyl-3-[3-(trifluoromethyl)phenoxy]-1-azetidinecarboxamide;
N-(1-methylethyl)-3-[3-(trifluoromethyl)phenoxy]-1-azetidinecarboxamide;
N-propyl-3-[3-(trifluoromethyl)phenoxy]-1-azetidinecarboxamide;
N-butyl-3-[3-(trifluoromethyl)phenoxy]-1-azetidinecarboxamide;

N-ethyl-3-[4-(trifluoromethyl)phenoxy]-1-azetidinecarboxamide;
N-butyl-3-[4-(trifluoromethyl)phenoxy]-1-azetidinecarboxamide;
N-propyl-3-[4-(trifluoromethyl)phenoxy]-1-azetidinecarboxamide;
N-(1-methylethyl)-3-[4-(trifluoromethyl)phenoxy]-1-azetidinecarboxamide;
N-(1,1-dimethylethyl)-3-[4-(trifluoromethyl)phenoxy]-1-azetidinecarboxamide;
N-(2-methylpropyl)-3-[3-(trifluoromethyl)phenoxy]-1-azetidinecarboxamide;
N-(1,1-dimethylethyl)-3-[3-(trifluoromethyl)phenoxy-1-azetidinecarboxamide;
N-(2-methylpropyl)-3-[4-(trifluoromethyl)phenoxy]-1-azetidinecarboxamide;
3-(3-chlorophenoxy)-1-azetidinecarboxamide;
3-(3-chlorophenoxy)-N-methyl-1-azetidinecarboxamide; or
3-(3-fluorophenoxy)-N-methyl-1-azetidinecarboxamide.

16. The use of a compound as defined in claim 11 in the preparation of an agent for treating muscle spasticity and/or muscle tension.

17. The use of a compound according to claim 11, wherein $R^1$ and/or $R^2$ represent(s) allyl, ($C_1$–$C_8$) alkyl substituted allyl, propargyl or cycloalkyl, in the preparation of an agent for treating muscle spasticity and/or muscle tension.

18. The use of a compound according to claim 11 in the preparation of an agent for treating anxiety.

**Patentansprüche**

1. Verbindung mit der allgemeinen Formel (I'),

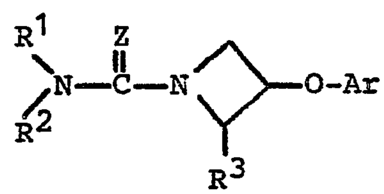

(I')

wobei
Ar Pyridil (in allen Positionen), durch Halo substituiertes Pyridil, Phenyl, oder durch 1 oder 2 Radikale wie Chlor, Brom, Iod, Fluor, ($C_1$–$C_8$)-Alkyl, ($C_1$–$C_8$)-Alkoxy, Nitro, Aminocarbonyl oder Trifluor-methylradikale substituiertes Phenyl darstellt;
Z Sauerstoff oder Schwefel darstellt;
sowohl $R^1$ als auch $R^2$ unabhängig voneinander Wasserstoff, ($C_1$–$C_8$)-Alkyl, Aryl, Allyl, durch ($C_1$–$C_8$)-Alkyl substituiertes Allyl, Propargyl, Cycloalkyl, ($C_1$–$C_8$)-Alkylcycloalkyl, Cycloalkyl-($C_1$–$C_8$)-Alkyl, Aryl-($C_1$–$C_8$)-Alkyl, und Di-($C_1$–$C_8$)-Alkylamino-($C_1$–$C_8$)-Alkyl zeigen, oder $R^1$ und $R^2$, wenn zusammengenommen mit dem benachbarten Stickstoffatom ein heterocyclisches Aminradikal bilden, beinhaltend Azetidinyl, Pyrrolidinyl, Piperidinyl, Homopiperidinyl, Imidazolyl, Piperazinyl, 4-substituiertes Piperazinyl und Morpholinyl;
$R^3$ Wasserstoff, ($C_1$–$C_8$)-Alkyl, Aryl oder ein pharmazeutisch akzeptables Säureadditionssalz davon darstellt, wenn $R^1$ und/oder $R^2$ einen salzbildenden Aminogrundbestandteil haben oder Ar Pyridil ist und sich der Begriff "Aryl" unter der Definition von $R^1$, $R^2$ und $R^3$ auf Phenyl oder Phenyl bezieht, das durch nichtstörende Radikale wie Halo, ($C_1$–$C_8$)-Alkyl, ($C_1$–$C_8$)-Alkoxy, Nitro, Cyano, Trifluor-methyl, Carbomethoxy oder Carboethoxy substituiert ist, und sich weiters der Begriff "Alkyl unter Aryl" unter der Definition von $R^1$, $R^2$ und $R^3$ auf eine oben definierte Arylgruppe bezieht, die mittels 1–8 Kohlenstoffalkylketten, inklusive verzweigter Ketten, mit dem Amidstickstoff verbunden ist;
unter der Voraussetzung, daß $R^3$ Wasserstoff ist, Z Sauerstoff ist und Ar Phenyl oder durch Trifluoromethyl oder Aminocarbonyl substituiertes Phenyl darstellt, $R^1$ und $R^2$ keine Kombination von Wasserstoff und ($C_1$–$C_8$)-Alkyl sein können, und unter der weiteren Voraussetzung, daß $R^3$ Wasserstoff ist, Z Sauerstoff ist und Ar Phenyl oder Phenyl, substituiert durch Fluor, ($C_1$–$C_8$)-Alkyl, ($C_1$–$C_8$)-Alkoxy, Trifluor-methyl, Acetyl oder durch Aminocarbonyl, darstellt, $R^1$ und $R^2$ nicht beide Wasserstoff sein können.

2. Verbindung nach Anspruch 1, wobei Ar durch Trifluor-methyl substituiertes Phenyl, 2-Pyridinyl oder durch Aminocarbonyl substituiertes Phenyl darstellt.

3. Verbindung nach Anspruch 1 oder 2, wobei $R^3$ Wasserstoff oder ($C_1$–$C_8$)-Alkyl darstellt.

4. Verbindung nach Anspruch 1, 2 oder 3, wobei zumindest eines von $R^1$ und $R^2$ Wasserstoff darstellt.

5. Verbindung nach einem der Ansprüche 1–4, wobei $R^1$ und/oder $R^2$ Allyl, durch ($C_1$–$C_8$)-Alkyl substituiertes Allyl, Propargyl oder Cycloalkyl sind.

6. N-Methyl-3-[3-(trifluor-methyl)phenoxy]-1-azetidincarbothioamid;
N-(2,6-Dimethylphenyl)-3-[3-(trifluor-methyl)phenoxy]-1-azetidincarbothioamid;
N-(Phenylmethyl)-3-[3-(trifluor-methyl)phenoxy]-1-azetidincarboxamid;
N-(2,6-Dichlorophenyl)-3-[3-(trifluor-methyl)phenoxy]-1-azetidincarbothioamid;
N-[3-(Diethylamino)propyl]-3-[3-(trifluor-methyl)phenoxy]-1-azetidincarbothioamid oder ein pharmazeutisch akzeptables Säureadditionssalz davon;

N-[3-(Dimethylamino)propyl]-3-[3-(trifluor-methyl)phenoxy]-1-azetidincarbothioamid oder ein pharmazeutisch akzeptables Säureadditionssalz davon;
N-(2-Propenyl)-3-[3-(trifluor-methyl)phenoxy]-1-azetidincarboxamid;
N-Cyclopropyl-3-[3-(trifluor-methyl)phenoxy]-1-azetidincarboxamid;
N-[3-(Diethylamino)propyl]-3-[3-(trifluor-methyl)phenoxy]-1-azetidincarboxamid oder ein pharmazeutisch akzeptables Säureadditionssalz davon;
N-2-(Propenyl)-3-[4-(trifluor-methyl)phenoxy]-1-azetidincarboxamid;
N-(Cyclopropylmethyl)-3-[3-(trifluor-methyl)phenoxy]-1-azetidincarboxamid;
N,N-Diethyl-3-[3-(trifluor-methyl)phenoxy]-1-azetidincarboxamid;
N,N-Dimethyl-3-[3-(trifluor-methyl)phenoxy]-1-azetidincarboxamid;
N-(2-Propinyl)-3-[3-(trifluor-methyl)phenoxy]-1-azetidincarboxamid;
N-Cyclohexyl-3-[3-(trifluor-methyl)phenoxy]-1-azetidincarboxamid;
N-Cyclopropyl-3-[4-(trifluor-methyl)phenoxy]-1-azetidincarboxamid;
N-(Cyclopropylmethyl)-3-[4-(trifluor-methyl)phenoxy]-1-azetidincarboxamid;
N-[3-(Diethylamino)propyl]-3-[4-(trifluor-methyl)phenoxy]-1-azetidincarbothioamid oder ein pharmazeutisch akzeptables Säureadditionssalz davon;
N-[3-(Diethylamino)propyl]-3-[4-(trifluor-methyl)phenoxy]-1-azetidincarboxamid oder ein pharmazeutisch akzeptables Säureadditionssalz davon;
N-(2-Propinyl)-3-[4-(trifluor-methyl)phenoxy]-1-azetidincarboxamid;
N-(2-Methyl-2-propenyl)-3-[4-(trifluor-methyl)phenoxy]-1-azetidincarboxamid;
N-(2-Methyl-2-propenyl)-3-[3-(trifluor-methyl)phenoxy]-1-azetidincarboxamid;
N-(3-Methyl-2-butenyl)-3-[4-(trifluor-methyl)phenoxy]-1-azetidincarboxamid;
N-(3-Methyl-2-butenyl)-3-[3-(trifluor-methyl)phenoxy]-1-azetidincarboxamid;
(E)-N-(2-Butenyl)-3-[4-(trifluor-methyl)phenoxy]-1-azetidincarboxamid;
(E)-N-(2-Butenyl)-3-[3-(trifluor-methyl)phenoxy]-1-azetidincarboxamid;
N-Phenyl-3-[3-(trifluor-methyl)phenoxy]-1-azetidincarboxamid;
N-Phenyl-3-[4-(trifluor-methyl)phenoxy]-1-azetidincarboxamid;
Trans-N,2-dimethyl-3-[3-(trifluor-methyl)phenoxy]-1-azetidincarboxamid;
Trans-2-methyl-3-[3-(trifluor-methyl)phenoxy]-1-azetidincarboxamid;
Trans-2-methyl-N-(2-propenyl)-3-[3-(trifluor-methyl)phenoxy]-1-azetidincarboxamid;
3-(3-Chlor-phenoxy)-N-methyl-1-azetidincarboxamid;
3-(3-Chlor-phenoxy)-N-(2-propenyl)-1-azetidincarboxamid;
N-Methyl-3-(2-pyridinyloxy)-1-azetidincarboxamid oder ein pharmazeutisch akzeptables Säureadditionssalz davon;
N-(2-Propenyl)-3-(2-pyridinyloxy)-1-azetidincarboxamid oder ein pharmazeutisch akzeptables Säureadditionssalz davon;
3-(2-Pyridinyloxy)-1-azetidincarboxamid oder ein pharmazeutisch akzeptables Säureadditionssalz davon;
2-[1-(1-Phenylethyl)-3-azetidinyloxy]pyridin oder ein pharmazeutisch akzeptables Säureadditionssalz davon;
1-[3-[4-(Trifluor-methyl)phenoxy]-1-azetidinylcarbonyl]-1H-imidazol oder ein pharmazeutisch akzeptables Säureadditionssalz davon; 3-(3-Chlor-phenoxy)-N-methyl-1-azetidincarboxamid; oder
3-(3-Fluor-phenoxy)-N-methyl-1-azetidincarboxamid.

7. Pharmazeutische Zusammensetzung, bestehend aus einer Verbindung, wie sie in einem der Ansprüche 1 bis 6 definiert ist, und einem pharmazeutisch akzeptablen Träger dafür.

8. Verbindung nach einem der Ansprüche 1 bis 6 zur Verwendung in der Human- und Veterinärmedizin.

9. Verwendung einer Verbindung nach einem der Ansprüche 1 bis 6 bei der Herstellung eines Mittels zur Anwendung bei der Behandlung oder der Prophylaxe von Epilepsie und/oder Krämpfen.

10. Verfahren für die Herstellung einer Verbindung, wie sie in den Ansprüchen 1 bis 6 definiert ist, wobei das Verfahren

(A) eine Reaktion beinhaltet zwischen einer Verbindung mit der allgemeinen Formel (II)

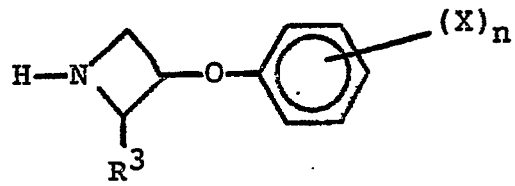

(II)

und einer der folgenden Klassen von Verbindungen:

a) $R^2N = C = Z$

b) 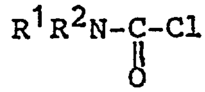

c) 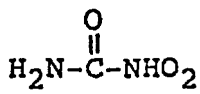

d) 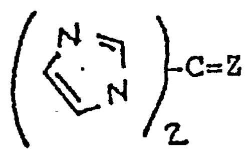

e) und f) das Produkt von 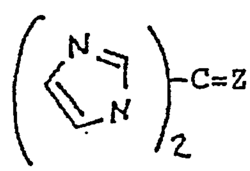

und $R^2NH_2$ oder 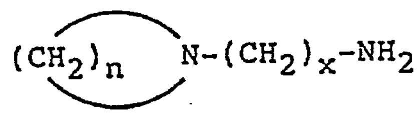

wobei $R^1$, $R^2$, $R^3$, Ar und Z die gleiche Bedeutung haben wie bei der allgemeinen Formel I' definiert ist, $(X)_n$ einen oder mehr Substituenten für Phenyl darstellt, wie bei der allgemeinen Formel I' definiert, und x eine ganze Zahl zwischen 0 und 8 ist; oder
(B) im Falle von bestimmten Aryloxyverbindungen mit der allgemeinen Formel I' eine Reaktion beinhaltet zwischen einer Verbindung mit der allgemeinen Formel (III)

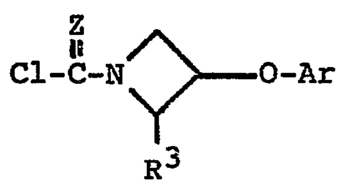

(III)

und einer der folgenden Klassen von Verbindungen:
a) $R^2NH_2$
b) heterocyclische Aminoverbindungen
wobei $R^2$, $R^3$, Ar und Z die gleiche Bedeutung haben wie bei der allgemeinen Formel I' definiert ist; oder
(C) im Falle von m-Fluor-phenoxyverbindungen mit der allgemeinen Formel I' eine Reaktion beinhaltet zwischen einer Verbindung mit der allgemeinen Formel (IV)

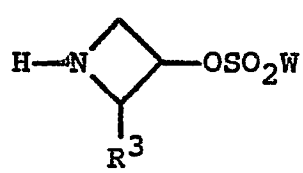

(IV)

wobei $R^3$ die gleiche Bedeutung hat wie bereits bei der allgemeinen Formel I' definiert ist, und W Methyl, Phenyl oder 4-Methylphenyl darstellt,
und einem Isozyanat mit der Formel

$$R^2{-}N{=}C{=}Z,$$

um eine Verbindung mit der Formel

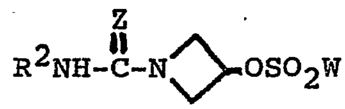

zu erhalten, und danach eine Reaktion beinhaltet zwischen Natriumhydrid und einem Metafluor-phenol mit der Formel

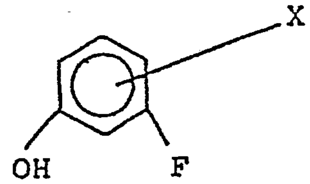

,

um eine Verbindung mit der Formel

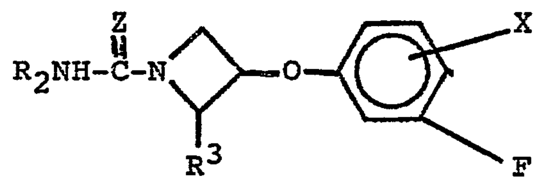

zu erhalten, wobei $R^2$, $R^3$ und Z die gleiche Bedeutung haben wie bei der allgemeinen Formel I′ definiert ist, und X einen oder mehr Substituenten, wie bei der allgemeinen Formel I′ definiert, darstellt.

11. Verwendung einer Verbindung mit der allgemeinen Formel I,

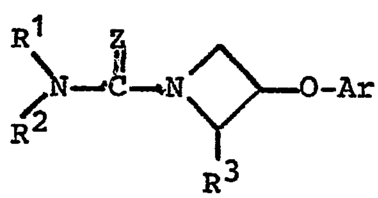

(I)

wobei

Ar Pyridil (in allen Positionen), durch Halo substituiertes Pyridil, Phenyl, oder durch 1 oder 2 Radikale wie Chlor, Brom, Iod, Fluor, $(C_1-C_8)$-Alkyl, $(C_1-C_8)$-Alkoxy, Nitro, Aminocarbonyl oder Trifluor-methylradikale substituiertes Phenyl darstellt;

Z Sauerstoff oder Schwefel darstellt;

sowohl $R^1$ als auch $R^2$ unabhängig voneinander Wasserstoff, $(C_1-C_8)$-Alkyl, Aryl, Allyl, durch $(C_1-C_8)$-Alkyl substituiertes Allyl, Propargyl, Cycloalkyl, $(C_1-C_8)$-Alkylcycloalkyl, Cycloalkyl-$(C_1-C_8)$-Alkyl, Aryl-$(C_1-C_8)$-Alkyl, und Di-$(C_1-C_8)$-Alkylamino-$(C_1-C_8)$-Alkyl zeigen, oder $R^1$ und $R^2$, wenn zusammengenommen mit dem benachbarten Stickstoffatom ein heterocyclisches Aminradikal bilden, beinhaltend Azetidinyl, Pyrrolidinyl, Piperidinyl, Homopiperidinyl, Imidazolyl, Piperazinyl, 4-substituiertes Piperazinyl und Morpholinyl;

$R^3$ Wasserstoff, $(C_1-C_8)$-Alkyl, Aryl oder ein pharmazeutisch akzeptables Säureadditionssalz davon darstellt, wenn $R^1$ und/oder $R^2$ einen salzbildenden Aminogrundbestandteil haben oder Ar Pyridil ist;

bei der Herstellung eines Mittels zur Behandlung von Muskelverspannung, Muskelspastizität und/oder Angstzuständen bei Tieren und Menschen, wobei das Mittel bei der Behandlung von Muskelverspannung und/oder Muskelspastizität in einer Dosis von 4–40 mg/kg pro Tag zu verabreichen ist.

12. Verwendung einer Verbindung nach Anspruch 11, wobei Ar durch Trifluor-methyl substituiertes Phenyl, 2-Pyridinyl oder durch Aminocarbonyl substituiertes Phenyl darstellt.

13. Verwendung einer Verbindung nach Anspruch 11 oder 12, wobei $R^3$ Wasserstoff oder $(C_1-C_8)$-Alkyl darstellt.

14. Verwendung einer Verbindung nach Anspruch 11, 12 oder 13, wobei mindestens eines von $R^1$ oder $R^2$ Wasserstoff darstellt.

15. Verwendung einer Verbindung nach Anspruch 11, nämlich
N-Methyl-3-[3-(trifluor-methyl)phenoxy]-1-azetidincarbothioamid;
N-(2,6-Dimethylphenyl)-3-[3-(trifluor-methyl)phenoxy]-1-azetidincarbothioamid;
N-(Phenylmethyl)-3-[3-(trifluor-methyl)phenoxy]-1-azetidincarboxamid;

N-(2,6-Dichlor-phenyl)-3-[3-(trifluor-methyl)phenoxy]-1-azetidincarbothioamid;
N-[3-(Diethylamino)propyl]-3-[3-(trifluor-methyl)phenoxy]-1-azetidincarbothioamid oder ein pharmazeutisch akzeptables Säureadditionssalz davon;
N-[3-(Dimethylamino)propyl]-3-[3-(trifluor-methyl)phenoxy]-1-azetidincarbothioamid oder ein pharmazeutisch akzeptables Säureadditionssalz davon;
N-(2-Propenyl)-3-[3-(trifluor-methyl)phenoxy]-1-azetidincarboxamid;
N-Cyclopropyl-3-[3-(trifluor-methyl)phenoxy]-1-azetidincarboxamid;
N-[3-(Diethylamino)propyl]-3-[3-(trifluor-methyl)phenoxy]-1-azetidincarboxamid oder ein pharmazeutisch akzeptables Säureadditionssalz davon;
N-2-(Propenyl)-3-[4-(trifluor-methyl)phenoxy]-1-azetidincarboxamid;
N-(Cyclopropylmethyl)-3-[3-(trifluor-methyl)phenoxy]-1-azetidincarboxamid;
N,N-Diethyl-3-[3-(trifluor-methyl)phenoxy]-1-azetidincarboxamid;
N,N-Dimethyl-3-[3-(trifluor-methyl)phenoxy]-1-azetidincarboxamid;
N-(2-Propinyl)-3-[3-(trifluor-methyl)phenoxy]-1-azetidincarboxamid;
N-Cyclohexyl-3-[3-(trifluor-methyl)phenoxy]-1-azetidincarboxamid;
N-Cyclopropyl-3-[4-(trifluor-methyl)phenoxy]-1-azetidincarboxamid;
N-(Cyclopropylmethyl)-3-[4-(trifluor-methyl)phenoxy]-1-azetidincarboxamid;
N-[3-(Diethylamino)propyl]-3-[4-(trifluor-methyl)phenoxy]-1-azetidincarbothioamid oder ein pharmazeutisch akzeptables Säureadditionssalz davon;
N-[3-(Diethylamino)propyl]-3-[4-(trifluor-methyl)phenoxy]-1-azetidincarboxamid oder ein pharmazeutisch akzeptables Säureadditionssalz davon;
N-(2-Propinyl)-3-[4-(trifluor-methyl)phenoxy]-1-azetidincarboxamid;
N-(2-Methyl-2-propenyl)-3-[4-(trifluor-methyl)phenoxy]-1-azetidincarboxamid;
N-(2-Methyl-2-propenyl)-3-[3-(trifluor-methyl)phenoxy]-1-azetidincarboxamid;
N-(3-Methyl-2-butenyl)-3-[4-(trifluor-methyl)phenoxy]-1-azetidincarboxamid;
N-(3-Methyl-2-butenyl)-3-[3-(trifluor-methyl)phenoxy]-1-azetidincarboxamid;
(E)-N-(2-Butenyl)-3-[4-(trifluor-methyl)phenoxy]-1-azetidincarboxamid;
(E)-N-(2-Butenyl)-3-[3-(trifluor-methyl)phenoxy]-1-azetidincarboxamid;
N-Phenyl-3-[3-(trifluor-methyl)phenoxy]-1-azetidincarboxamid;
N-Phenyl-3-[4-(trifluor-methyl)phenoxy]-1-azetidincarboxamid;
Trans-N,2-dimethyl-3-[3-(trifluor-methyl)phenoxy]-1-azetidincarboxamid;
Trans-2-methyl-3-[3-(trifluor-methyl)phenoxy]-1-azetidincarboxamid;
Trans-2-methyl-N-(2-propenyl)-3-[3-(trifluor-methyl)phenoxy]-1-azetidincarboxamid;
3-(3-Chlor-phenoxy)-N-methyl-1-azetidincarboxamid;
3-(3-Chlor-phenoxy)-N-(2-propenyl)-1-azetidincarboxamid;
N-Methyl-3-(2-pyridinyloxy)-1-azetidincarboxamid oder ein pharmazeutisch akzeptables Säureadditionssalz davon;
N-(2-Propenyl)-3-(2-pyridinyloxy)-1-azetidincarboxamid oder ein pharmazeutisch akzeptables Säureadditionssalz davon;
3-(2-Pyridinyloxy)-1-azetidincarboxamid oder ein pharmazeutisch akzeptables Säureadditionssalz davon;
2-[1-(1-Phenylethyl)-3-azetidinyloxy]pyridin oder ein pharmazeutisch akzeptables Säureadditionssalz davon;
1-[3-[4-(Trifluor-methyl)phenoxy]-1-azetidinylcarbonyl]-1H-imidazol oder ein pharmazeutisch akzeptables Säureadditionssalz davon;
N-Methyl-3-phenoxy-1-azetidincarboxamid;
N-Methyl-3-[4-(trifluor-methyl)phenoxy]-1-azetidincarboxamid;
N-Methyl-3-[3-(trifluor-methyl)phenoxy]-1-azetidincarboxamid;
N-Methyl-3-[2-(trifluor-methyl)phenoxy]-1-azetidincarboxamid;
N-Methyl-3-[2-(aminocarbonyl)phenoxy]-1-azetidincarboxamid;
N-Methyl-3-[3-(aminocarbonyl)phenoxy]-1-azetidincarboxamid;
N-Methyl-3-[4-(aminocarbonyl)phenoxy]-1-azetidincarboxamid;
3-[3-(Trifluor-methyl)phenoxy]-1-azetidincarboxamid;
N-Ethyl-3-[3-(trifluor-methyl)phenoxy]-1-azetidincarboxamid;
N-(1-Methylethyl)-3-[3-(trifluor-methyl)phenoxy]-1-azetidincarboxamid;
N-Propyl-3-[3-(trifluor-methyl)phenoxy]-1-azetidincarboxamid;
N-Butyl-3-[3-(trifluor-methyl)phenoxy]-1-azetidincarboxamid;
N-Ethyl-3-[4-(trifluor-methyl)phenoxy]-1-azetidincarboxamid;
N-Butyl-3-[4-(trifluor-methyl)phenoxy]-1-azetidincarboxamid;
N-Propyl-3-[4-(trifluor-methyl)phenoxy]-1-azetidincarboxamid;
N-(1-Methylethyl)-3-[4-(trifluor-methyl)phenoxy]-1-azetidincarboxamid;
N-(1,1-Dimethylethyl)-3-[4-(trifluor-methyl)phenoxy]-1-azetidincarboxamid;
N-(2-Methylpropyl)-3-[3-(trifluor-methyl)phenoxy]-1-azetidincarboxamid;
N-(1,1-Dimethylethyl)-3-[3-(trifluor-methyl)phenoxy]-1-azetidincarboxamid;
N-(2-Methylpropyl)-3-[4-(trifluor-methyl)phenoxy]-1-azetidincarboxamid;

3-(3-Chlor-phenoxy)-1-azetidincarboxamid;
3-(3-Chlor-phenoxy)-N-methyl-1-azetidincarboxamid; oder
3-(3-Fluor-phenoxy)-N-methyl-1-azetidincarboxamid.

16. Verwendung einer Verbindung nach Anspruch 11 bei der Herstellung eines Mittels zur Behandlung von Muskelspastizität und/oder Muskelverspannung.

17. Verwendung einer Verbindung nach Anspruch 11, wobei $R^1$ und/oder $R^2$ Allyl, durch ($C_1$–$C_8$)-Alkyl substituiertes Allyl, Propargyl oder Cycloalkyl darstellen, bei der Herstellung eines Mittels zur Behandlung von Muskelspastizität und/oder Muskelverspannung.

18. Verwendung einer Verbindung nach Anspruch 11 bei der Herstellung eines Mittels zur Behandlung von Angstzuständen.

**Revendications**

1. Composé de formule générale I'

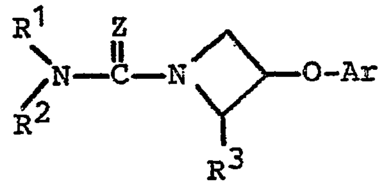

(I')

où Ar représente pyridyle (dans l'une quelconque de ses positions), pyridyle substitué par halo, phényle ou phényle substitué par 1 ou 2 radicaux chloro, bromo, iodo, fluoro, ($C_1$–$C_8$)-alcoyle, ($C_1$–$C_8$)-alcoxy, nitro, aminocarbonyle ou trifluorométhyle;
Z représente oxygène ou soufre; chacun d'entre $R^1$ et $R^2$ représente indépendamment hydrogène, ($C_1$–$C_8$)-alcoyle, aryle, allyle, allyle ($C_1$–$C_8$)-alcoyl-substitué, propargyle, cycloalcoyle, ($C_1$–$C_8$)-alcoyl-cycloalcoyle, cycloalcoyl-($C_1$–$C_8$)-alcoyle, aryl-($C_1$–$C_8$)-alcoyle et di-($C_1$–$C_8$)-alcoylamino-($C_1$–$C_8$)-alcoyle, ou bien $R^1$ et $R^2$, pris ensemble avec l'atome d'azote adjacent, forment un radical amino hétérocyclique notamment azétidinyle, pyrrolidinyle, pipéridinyle, homopipéridinyle, imidazolyle, pipérazinyle, pipérazinyle 4-substitué et morpholinyle;
$R^3$ représente hydrogène, ($C_1$–$C_8$)-alcoyle ou aryle, ou un sel d'addition d'acide pharmaceutiquement acceptable de celui-ci lorsque $R^1$ et/ou $R^2$ ont une fonction amino basique formant un sel ou bien Ar est pyridyle et le terme "aryle" sous la définition de $R^1$, $R^2$ et $R^3$ désignant phényle ou phényle substitué par des radicaux non gênants tels que halo, ($C_1$–$C_8$)-alcoyle, ($C_1$–$C_8$)-alcoxy, nitro, cyano, trifluorométhyle, carbométhoxy ou carboéthoxy,
et de surcroît le terme "arylalcoyle inférieur" sous la définition de $R^1$, $R^2$ et $R^3$ désignant un radical aryle tel que défini ci-dessus uni à l'azote d'amide par des chaînes alcoyle, y compris les chaînes ramifiées, de 1 à 8 atomes de carbone,
avec la restriction que lorsque $R^3$ représente hydrogène, Z représente oxygène et Ar représente phényle ou phényle substitué par trifluorométhyle ou par aminocarbonyle, alors $R^1$ et $R^2$ ne peuvent être une combinaison hydrogène et ($C_1$–$C_8$)-alcoyle et avec la restriction supplémentaire que lorsque $R^3$ représente hydrogène, Z représente oxygène et Ar représente phényle ou phényle substitué par fluoro, par ($C_1$–$C_8$)-alcoyle, par ($C_1$–$C_8$)-alcoxy, par trifluorométhyle, par acétyle ou par aminocarbonyle, alors $R^1$ et $R^2$ ne peuvent être tous deux hydrogène.

2. Composé suivant la revendication 1, dans lequel Ar représente phényle trifluorométhyl-substitué, 2-pyridinyle ou phényle aminocarbonyl-substitué.

3. Composé suivant la revendication 1 ou 2, dans lequel $R^3$ représente hydrogène ou ($C_1$–$C_8$)-alcoyle.

4. Composé suivant la revendication 1, 2 ou 3, dans lequel au moins l'un d'entre $R^1$ et $R^2$ représente hydrogène.

5. Composé suivant l'une quelconque des revendications 1 à 4, dans lequel $R^1$ et/ou $R^2$ représentent allyle, allyle ($C_1$–$C_8$)-alcoyl-substitué, propargyle ou cycloalcoyle.

6. Le N-méthyl-3-[3-(trifluorométhyl)phénoxy]-1-azétidinecarbothioamide;
le N-(2,6-diméthylphényl)-3-[3-(trifluorométhyl)phénoxy]-1-azétidinecarbothioamide;
le N-(phénylméthyl)-3-[3-(trifluorométhyl)phénoxy]-1-azétidinecarboxamide;
le N-(2,6-dichlorophényl)-3-[3-(trifluorométhyl)phénoxy]-1-azétidinecarbothioamide;
le N-[3-(diéthylamino)propyl]-3-[3-(trifluorométhyl)phénoxy]-1-azétidinecarbothioamide ou un sel d'addition d'acide pharmaceutiquement acceptable de celui-ci;
le N-[3-(diméthylamino)propyl]-3-[3-trifluorométhyl)phénoxy]-1-azétidinecarbothioamide ou un sel d'addition d'acide pharmaceutiquement acceptable de celui-ci;
le N-(2-propényl)-3-[3-(trifluorométhyl)phénoxy]-1-azétidinecarboxamide;
le N-cyclopropyl-3-[3-(trifluorométhyl)phénoxy]-1-azétidinecarboxamide;
le N-[3-(diéthylamino)propyl]-3-[3-(trifluorométhyl)phénoxy]-1-azédinecarboxamide ou un sel d'addition

d'acide pharmaceutiquement acceptable de celui-ci;
le N-2-(propényl)-3-[4-(trifluorométhyl)phénoxy-1-azétidinecarboxamide;
le N-(cyclopropylméthyl)-3-[3-(trifluorométhyl)phénoxy]-1-azétidinecarboxamide;
le N,N-diéthyl-3-[3-(trifluorométhyl)phénoxy]-1-azétidinecarboxamide;
le N,N-diméthyl-3-[3-(trifluorométhyl)phénoxy]-1-azétidinecarboxamide;
le N-(2-propynyl)-3-[3-(trifluorométhyl)phénoxy]-1-azétidinecarboxamide;
le N-cyclohexyl-3-[3-(trifluorométhyl)phénoxy]-1-azétidinecarboxamide;
le N-cyclopropyl-3-[4-(trifluorométhyl)phénoxy]-1-azétidinecarboxamide;
le N-(cyclopropylméthyl)-3-[4-(trifluorométhyl)phénoxy]-1-azétidinecarboxamide;
le N-[3-(diéthylamino)propyl]-3-[4-(trifluorométhyl)phénoxy]-1-azétidinecarbothioamide ou un sel d'addition d'acide pharmaceutiquement acceptable de celui-ci;
le N-[3-(diéthylamino)propyl]-3-[4-(trifluorométhyl)phénoxy]-1-azétidinecarboxamide ou un sel d'addition d'acide pharmaceutiquement acceptable de celui-ci;
le N-(2-propynyl)-3-[4-(trifluorométhyl)phénoxy]-1-azétidinecarboxamide;
le N-(2-méthyl-2-propényl)-3-[4-(trifluorométhyl)phénoxy]-1-azétidinecarboxamide;
le N-(2-méthyl-2-propényl)-3-[3-(trifluorométhyl)phénoxy]-1-azétidinecarboxamide;
le N-(3-méthyl-2-butényl)-3-[4-(trifluorométhyl)phénoxy]-1-azétidinecarboxamide;
le N-(3-méthyl-2-butényl)-3-[3-(trifluorométhyl)phénoxy]-1-azétidinecarboxamide;
le (E)-N-(2-butényl)-3-[4-trifluorométhyl)phénoxy]-1-azétidinecarboxamide;
le (E)-N-(2-butényl)-3-[3-(trifluoromethyl)phénoxy]-1-azétidinecarboxamide;
le N-phényl-3-[3-(trifluorométhyl)phénoxy]-1-azétidinecarboxamide;
le N-phényl-3-[4-(trifluorométhyl)phénoxy]-1-azétidinecarboxamide;
le trans-N,2-diméthyl-3-[3-(trifluorométhyl)phénoxy]-1-azétidinecarboxamide;
le trans-2-méthyl-3-[3-(trifluorométhyl)phénoxy]-1-azétidinecarboxamide;
le trans-2-méthyl-N-(2-propényl)-3-[3-(trifluorométhyl)phénoxy]-1-azétidinecarboxamide;
le 3-(3-chlorphénoxy)-N-méthyl-1-azétidinecarboxamide;
le 3-(3-chlorophénoxy)-N-(2-propényl)-1-atétidinecarboxamide;
le N-méthyl-3-(2-pyridinyloxy)-1-azétidinecarboxamide ou un sel d'addition d'acide pharmaceutiquement acceptable de celui-ci;
le N-(2-propényl)-3-(2-pyridinyloxy)-1-azétidinecarboxamide ou un sel d'addition d'acide pharmaceutiquement acceptable de celui-ci;
le 3-(2-pyridinyloxy)-1-azétidinecarboxamide ou un sel d'addition d'acide pharmaceutiquement acceptable de celui-ci;
la 2-[1-(1-phényléthyl)-3-azétidinyloxy]pyridine ou un sel d'addition d'acide pharmaceutiquement acceptable de celle-ci;
le 1-[3-[4-(trifluorométhyl)phénoxy]-1-azétidinylcarbonyl]-1H-imidazole ou un sel d'addition d'acide pharmaceutiquement acceptable de celui-ci;
le 3-(3-chlorophénoxy)-N-méthyl-1-azétidinecarboxamide; ou
le 3-(3-fluorophénoxy)-N-méthyl-1-azétidinecarboxamide.

7. Composition pharmaceutique, comprenant un composé tel que défini dans l'une quelconque des revendications 1 à 6 et un excipient pharmaceutiquement acceptable.

8. Composé tel que défini dans l'une quelconque des revendications 1 à 6, à utiliser un médecine humaine ou vétérinaire.

9. Utilisation d'un composé tel que défini dans l'une quelconque des revendications 1 à 6 dans la préparation d'un agent à utiliser pour le traitement ou la prophylaxie de l'épilepsie et/ou des convulsions.

10. Procédé de préparation d'un composé tel que défini dans l'une quelconque des revendications 1 à 6, lequel procédé comprend
soit (A) la réaction d'un composé de formule générale (II)

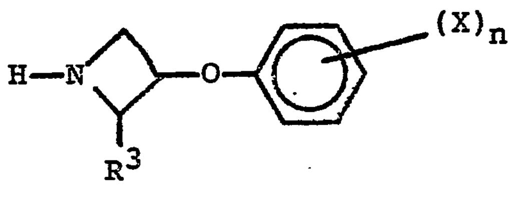

(II)

avec l'une des classes de composés suivantes:

a) $R^2N = C = Z$

b) 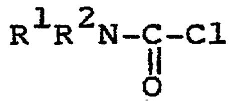

c) 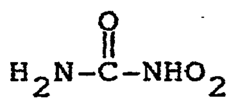

d) 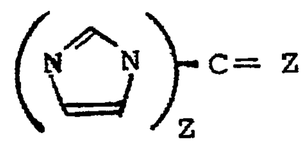

e) et f) le produit de

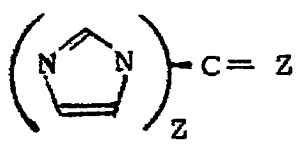

et $R^2NH_2$ ou 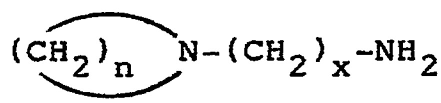

où R¹, R², R³, Ar et Z sont tels que définis à propos de la formule générale I′, $(X)_n$ représente un ou plusieurs substituants pour phényle tels que définis à propos de la formule générale I′ et x est un entier de 0 à 8;

soit (B), dans le cas de certains aryloxy-composés de formule générale I′, la réaction d'un composé de formule générale (III)

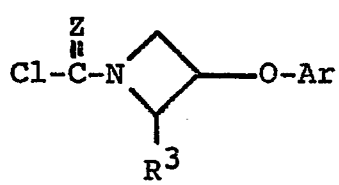

(III)

avec l'une des classes de composés suivantes:

a) $R^2NH_2$

b) des composés aminés hétérocycliques où R², R³, Ar et Z sont tels que définis à propos de la formule générale I′;

soit (C), dans le cas de m-fluorophénoxy-composés de formule générale I′, la réaction d'un composé de formule générale (IV)

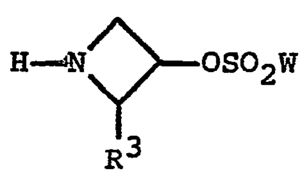

(IV)

où R³ est tel que défini à propos de la formule générale I′ et W représente méthyle, phényle ou 4-méthylphényle, avec un isocyanate de formule

$R^2–N=C=Z$

pour la formation d'un composé de formule

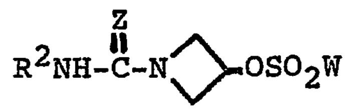

et ensuite la réaction avec l'hydrure de sodium et un métafluorophénol de formule

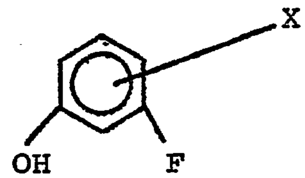

pour la formation d'un composé de formule

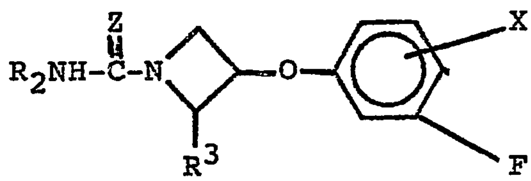

où $R^2$, $R^3$ et Z sont tels que définis à propos de la formule générale I' ex X représente un ou plusieurs substituants tels que définis à propos de la formule générale I'.

11. Utilisation d'un composé de formule générale I:

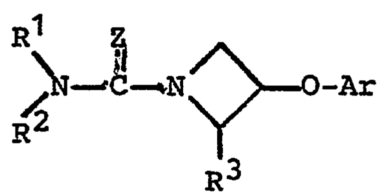

(I)

où Ar représente pyridyle (dans l'une quelconque de ses positions), pyridyle substitué par halo, phényle ou phényle substitué par 1 ou 2 radicaux chloro, bromo, iodo, fluoro, $(C_1–C_8)$-alcoyle, $(C_1–C_8)$-alcoxy, nitro, aminocarbonyle ou trifluorométhyle;
Z représente oxygène ou soufre;
chacun d'entre $R^1$ et $R^2$ représente indépendamment hydrogène, $(C_1–C_8)$-alcoyle, aryle, allyle, allyle $(C_1–C_8)$-alcoyl-substitué, propargyle, cycloalcoyle, $(C_1–C_8)$-alcoylcycloalcoyle, cycloalcoyl-$(C_1–C_8)$-alcoyle, aryl-$(C_1–C_8)$-alcoyle et di-$(C_1–C_8)$-alcoylamino-$(C_1–C_8)$-alcoyle, ou bien $R^1$ et $R^2$, pris ensemble avec l'atome d'azote adjacent, forment un radical amino hétérocyclique notamment azétidinyle, pyrrolidinyle, pipéridinyle, homopipéridinyle, imidazolyle, pipérazinyle, pipérazinyle 4-substitué et morpholinyle;
$R^3$ représente hydrogène, $(C_1–C_8)$-alcoyle ou aryle, ou d'un sel d'addition d'acide pharmaceutiquement acceptable de celui-ci lorsque $R^1$ et/ou $R^2$ ont une fonction amino basique formant un sel ou bien Ar est pyridyle; dans la préparation d'un agent pour le traitement de la tension musculaire, de la spasticité musculaire et/ou de l'anxiété chez les animaux, y compris l'être humain, où pour le traitement de la tension musculaire et/ou de la spasticité musculaire, l'agent est à administrer en une dose de 4 à 40 mg/kg et par jour.

12. Utilisation d'un composé suivant la revendication 11, où Ar représente phényle trifluorométhyl-substitué, 2-pyridinyle ou phényle aminocarbonyl-substitué.

13. Utilisation d'un composé suivant la revendication 11 ou 12, où $R^3$ représente hydrogène ou $(C_1–C_8)$-alcoyle.

14. Utilisation d'un composé suivant la revendication 11, 12 ou 13, où au moins l'une d'entre $R^1$ et $R^2$ représente hydrogène.

15. Utilisation d'un composé suivant la revendication 11 qui est
le N-méthyl-3-[3-(trifluorométhyl)phénoxy]-1-azétidinecarbothioamide;
le N-(2,6-diméthylphényl)-3-[3-(trifluorométhyl)phénoxy]-1-azétidinecarbothioamide;
le N-(phénylméthyl)-3-[3-(trifluorométhyl)phénoxy]-1-azétidinecarboxamide;

le N-(2,6-dichlorophényl)-3-[3-(trifluorométhyl)phénoxy]-1-azétidinecarbothioamide;
le N-[3-(diéthylamino)propyl]-3-[3-(trifluorométhyl)phénoxy]-1-azétidinecarbothioamide ou un sel d'addition d'acide pharmaceutiquement acceptable de celui-ci;
le N-[3-(diméthylamino)propyl]-3-[3-(trifluorométhyl)phénoxy]-1-azétidinecarbothioamide ou un sel d'addition d'acide pharmaceutiquement acceptable de celui-ci;
le N-(2-propényl)-3-[3-(trifluorométhyl)phénoxy]-1-azétidinecarboxamide;
le N-cyclopropyl-3-[3-(trifluorométhyl)phénoxy]-1-azétidinecarboxamide;
le N-[3-(diéthylamino)propyl]-3-[3-(trifluorométhyl)phénoxy]-1-azétidinecarboxamide ou un sel d'addition d'acide pharmaceutiquement acceptable de celui-ci;
le N-2-(propényl)-3-[4-(trifluorométhyl)phénoxy]-1-azétidinecarboxamide;
le N-(cyclopropylméthyl)-3-[3-(trifluorométhyl)phénoxy]-1-azétidinecarboxamide;
le N,N-diéthyl-3-[3-(trifluorométhyl)phénoxy]-1-azétidinecarboxamide;
le N,N-diméthyl-3-[3-(trifluorométhyl)phénoxy]-1-azétidinecarboxamide;
le N-(2-propynyl)-3-[3-(trifluorométhyl)phénoxy]-1-azétidinecarboxamide;
le N-cyclohexyl-3-[3-(trifluorométhyl)phénoxy]-1-azétidinecarboxamide;
le N-cyclopropyl-3-[4-(trifluorométhyl)phénoxy]-1-azétidinecarboxamide;
le N-(cyclopropylméthyl)-3-[4-(trifluorométhyl)phénoxy]-1-azétidinecarboxamide;
le N-[3-(diéthylamino)propyl]-3-[4-(trifluorométhyl)phénoxy]-1-azétidinecarbothioamide ou un sel d'addition d'acide pharmaceutiquement acceptable de celui-ci;
le N-[3-(diéthylamino)propyl]-3-[4-(trifluorométhyl)phénoxy]-1-azétidinecarboxamide ou un sel d'addition d'acide pharmaceutiquement acceptable de celui-ci;
le N-(2-propynyl)-3-[4-(trifluorométhyl)phénoxy]-1-azétidinecarboxamide;
le N-(2-méthyl-2-propényl)-3-[4-(trifluorométhyl)phénoxy]-1-azétidinecarboxamide;
le N-(2-méthyl-2-propényl)-3-[3-(trifluorométhyl)phénoxy]-1-azétidinecarboxamide;
le N-(3-méthyl-2-butényl)-3-[4-(trifluorométhyl)phénoxy]-1-azétidinecarboxamide;
le N-(3-méthyl-2-butényl)-3-[3-(trifluorométhyl)phénoxy]-1-azétidinecarboxamide;
le (E)-N-(2-butényl)-3-[4-(trifluorométhyl)phénoxy]-1-azétidinecarboxamide;
le (E)-N-(2-butényl)-3-[3-(trifluorométhyl)phénoxy]-1-azétidinecarboxamide;
le N-phényl-3-[3-(trifluorométhyl)phénoxy]-1-azétidinecarboxamide;
le N-phényl-3-[4-(trifluorométhyl)phénoxy]-1-azétidinecarboxamide;
le trans-N,2-diméthyl-3-[3-(trifluorométhyl)phénoxy]-1-azétidinecarboxamide;
le trans-2-méthyl-3-[3-(trifluorométhyl)phénoxy]-1-azétidinecarboxamide;
le trans-2-méthyl-N-(2-propényl)-3-[3-(trifluorométhyl)phénoxy]-1-azétidinecarboxamide;
le 3-(3-chlorophénoxy)-N-méthyl-1-azétidinecarboxamide;
le 3-(3-chlorophénoxy)-N-(2-propényl)-1-azétidinecarboxamide;
le N-méthyl-3-(2-pyridinyloxy)-1-azétidinecarboxamide ou un sel d'addition d'acide pharmaceutiquement acceptable de celui-ci;
le N-(2-propényl)-3-(2-pyridinyloxy)-1-azétidinecarboxamide ou un sel d'addition d'acide pharmaceutiquement acceptable de celui-ci;
le 3-(2-pyridinyloxy)-1-azétidinecarboxamide ou un sel d'addition d'acide pharmaceutiquement acceptable de celui-ci;
la 2-[1-(1-phényléthyl)-3-azétidinyloxy]-pyridine ou un sel d'addition d'acide pharmaceutiquement acceptable de celle-ci;
le 1-[3-[4-(trifluorométhyl)phénoxy]-1-azétidinylcarbonyl]-1H-imidazole ou un sel d'addition d'acide pharmaceutiquement acceptable de celui-ci;
le N-méthyl-3-phénoxy-1-azétidinecarboxamide;
le N-méthyl-3-[4-(trifluorométhyl)phénoxy]-1-azétidinecarboxamide;
le N-méthyl-3-[3-(trifluorométhyl)phénoxy]-1-azétidinecarboxamide;
le N-méthyl-3-[2-(trifluorométhyl)phénoxy]-1-azétidinecarboxamide;
le N-méthyl-3-[2-(aminocarbonyl)phénoxy]-1-azétidinecarboxamide;
le N-méthyl-3-[3-(aminocarbonyl)phénoxy]-1-azétidinecarboxamide;
le N-méthyl-3-[4-(aminocarbonyl)phénoxy]-1-azétidinecarboxamide;
le 3-[3-(trifluorométhyl)phénoxy]-1-azétidinecarboxamide;
le N-éthyl-3-[3-(trifluorométhyl)phénoxy]-1-azétidinecarboxamide;
le N-(1-méthyléthyl)-3-[3-(trifluorométhyl)phénoxy]-1-azétidinecarboxamide;
le N-propyl-3-[3-(trifluorométhyl)phénoxy]-1-azétidinecarboxamide;
le N-butyl-3-[3-(trifluorométhyl)phénoxy]-1-azétidinecarboxamide;
le N-éthyl-3-[4-(trifluorométhyl)phénoxy]-1-azétidinecarboxamide;
le N-butyl-3-[4-(trifluorométhyl)phénoxy]-1-azétidinecarboxamide;
le N-propyl-3-[4-(trifluorométhyl)phénoxy]-1-azétidinecarboxamide;
le N-(1-méthyléthyl)-3-[4-(trifluorométhyl)phénoxy]-1-azétidinecarboxamide;
le N-(1,1-diméthyléthyl)-3-[4-(trifluorométhyl)phénoxy]-1-azétidinecarboxamide;
le N-(2-méthylpropyl)-3-[3-(trifluorométhyl)phénoxy]-1-azétidinecarboxamide;
le N-(1,1-diméthyléthyl)-3-[3-(trifluorométhyl)phénoxy]-1-azétidinecarboxamide;
le N-(2-méthylpropyl)-3-[4-(trifluorométhyl)phénoxy]-1-azétidinecarboxamide;

le 3-(3-chlorophénoxy)-1-azétidinecarboxamide;
le 3-(3-chlorophénoxy)-N-méthyl-1-azétidinecarboxamide; ou
le 3-(3-fluorophénoxy)-N-méthyl-1-azétidinecarboxamide.

16. Utilisation d'un composé tel que défini dans la revendication 11 dans la préparation d'un agent pour le traitement de la spasticité musculaire et/ou de la tension musculaire.

17. Utilisation d'un composé suivant la revendication 11 où $R^1$ et/ou $R^2$ représentent allyle, allyle ($C_1$–$C_8$)-alcoyl-substitué, propargyle ou cycloalcoyle, dans la préparation d'un agent pour le traitement de la spasticité musculaire et/ou de la tension musculaire.

18. Utilisation d'un composé suivant la revendication 11, dans la préparation d'un agent pour le traitement de l'anxiété.